(19) Europäisches Patentamt
European Patent Office
Office européen des brevets

(11) Veröffentlichungsnummer: **0 321 385 B1**

# (12) EUROPÄISCHE PATENTSCHRIFT

(45) Veröffentlichungstag der Patentschrift: **17.08.94**

(51) Int. Cl.⁵: **C07D 495/04**, A61K 31/44, //(C07D495/04,333:00,221:00)

(21) Anmeldenummer: **88710043.6**

(22) Anmeldetag: **04.11.88**

## (54) Substituierte Thienoimidazol-Derivate, Verfahren zu ihrer Herstellung, sie enthaltende pharmazeutische Zubereitungen und ihre Verwendung als Magensäuresekretionshemmer.

(30) Priorität: **13.11.87 DE 3738520**
**04.06.88 DE 3819084**
**19.08.88 DE 3828158**

(43) Veröffentlichungstag der Anmeldung:
**21.06.89 Patentblatt 89/25**

(45) Bekanntmachung des Hinweises auf die Patenterteilung:
**17.08.94 Patentblatt 94/33**

(84) Benannte Vertragsstaaten:
**AT BE CH DE ES FR GB GR IT LI LU NL SE**

(56) Entgegenhaltungen:
**EP-A- 0 167 943**
**EP-A- 0 234 485**
**EP-A- 0 237 248**

(73) Patentinhaber: **HOECHST AKTIENGESELLSCHAFT**

**D-65926 Frankfurt (DE)**

(72) Erfinder: **Lang, Hans Jochen, Dr.**
**Rüdesheimer Strasse 7**
**D-6238 Hofheim am Taunus (DE)**
Erfinder: **Weidmann, Klaus, Dr.**
**Talweg 11**
**D-6242 Kronberg/Taunus (DE)**
Erfinder: **Scheunemann, Karl-Heinz, Dr.**
**Geisenheimer Strasse 88**
**D-6000 Frankfurt am Main 71 (DE)**
Erfinder: **Nimmesgern, Hildegard, Dr.**
**Rauenthaler Weg 32**
**D-6000 Frankfurt am Main 71 (DE)**
Erfinder: **Rippel, Robert, Dr.**
**Frankfurter Strasse 66**
**D-6238 Hofheim am Taunus (DE)**
Erfinder: **Herling, Andreas W.**
**Am Walberstück 5**
**D-6277 Bad Camberg (DE)**

EP 0 321 385 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Thienoimidazol-Derivate mit magensäuresekretionshemmender Wirkung sind aus EP-A1-234 485, EP-A2-201 094 und EP-A-237 248 bekannt.

Die vorliegende Erfindung betrifft neue Thienoimidazol-Derivate der Formel I

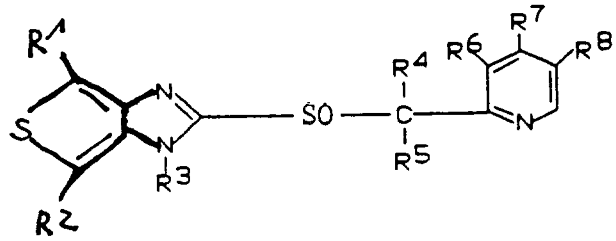

(I)

in welcher

- $R^1$ und $R^2$ gleich oder verschieden sind und Wasserstoff, $(C_1$-$C_3)$-Alkyl, Halogen, $(C_1$-$C_4)$-Alkoxy, $-O-[CH_2-]_xC_fH_{(2f+1-g)}F_g$ oder $(C_1$-$C_4)$-Alkoxycarbonyl bedeuten,
- $R^3$ Wasserstoff, $(C_1$-$C_6)$-Alkanoyl, $(C_1$-$C_6)$-Alkylcarbamoyl, $(C_1$-$C_6)$-Alkoxycarbonyl, Benzyloxycarbonyl oder eine andere physiologisch verträgliche, vorzugsweise im sauren Medium und/oder unter physiologischen Bedingungen abspaltbare $N^{im}$-Schutzgruppe wie z. B. $(C_1$-$C_{10})$-Acyloxy-$(C_1$-$C_6)$-alkyl, vorzugsweise $(C_1$-$C_{10})$-Alkanoyloxy-$(C_1$-$C_6)$-alkyl, Benzoyloxy-$(C_1$-$C_6)$-alkyl, Benzyloxycarbonyloxy-$(C_1$-$C_6)$-alkyl oder $(C_1$-$C_6)$-Alkoxycarbonyloxy-$(C_1$-$C_6)$-alkyl bedeutet,
- $R^4$ und $R^5$ jeweils Wasserstoff bedeuten,
- $R^6$ und $R^8$ gleich oder verschieden sind und Wasserstoff, Halogen, $(C_1$-$C_3)$-Alkyl, $(C_1$-$C_6)$-Alkoxy und einen Fluoralkoxy-Rest der Formel $-O-[CH_2-]_xC_fH_{(2f+1-g)}F_g$ bedeutet,
- $R^7$ einen ein- oder mehrfach substituierten Benzyloxy-oder Phenoxy-Rest der Formel

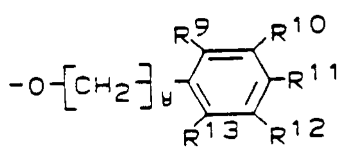

bedeutet, worin

- $R^9$, $R^{10}$, $R^{11}$, $R^{12}$ und $R^{13}$ gleich oder verschieden sind und Wasserstoff, Fluor, Chlor, Brom, Trifluormethyl, Cyano, Nitro, $(C_1$-$C_6)$-Alkoxycarbonyl bedeuten, und
- y 0 oder 1,
- f 1, 2, 3, 4, 5, 6, 7 oder 8, vorzugsweise 1 bis 5,
- g 0, 1 bis (2f + 1) ist und
- x 0, 1, 2 oder 3, vorzugsweise 0 oder 1 ist,

sowie deren physiologisch verträgliche Salze.

Insbesondere bevorzugt sind Verbindungen der Formel I, worin

- A wie oben unter (b) definiert ist,
- T eine -SO-Gruppe bedeutet,
- $R^1$ und $R^2$ gleich oder verschieden sind und Wasserstoff oder $(C_1$-$C_3)$-Alkyl, bedeuten,
- $R^3$ wie oben definiert ist,
- $R^4$ und $R^5$ jeweils Wasserstoff bedeuten,
- $R^6$ und $R^8$ gleich oder verschieden sind und Wasserstoff, $(C_1$-$C_3)$-Alkyl oder $(C_1$-$C_3)$-Alkoxy bedeuten,
- $R^7$ einen ein- oder mehrfach substituierten Benzyloxy-oder Phenoxy-Rest bedeutet,
- $R^9$, $R^{10}$, $R^{11}$, $R^{12}$ und $R^{13}$ gleich oder verschieden sind und Wasserstoff, Fluor, Chlor, Trifluormethyl

bedeuten und die übrigen Reste und Variablen wie oben definiert sind.

Von besonderer Bedeutung sind:

2-[4-(4-Trifluormethylbenzyloxy)-2-picolyl-sulfinyl]-1H-thieno[3,4-d]imidazol
2-[3-Methoxy-4-(4-trifluormethylbenzyloxy)-2-picolylsulfinyl]-1H-thieno[3,4-d]imidazol
2-[3-Methoxy-4-(4-fluorbenzyloxy)-2-picolylsulfinyl]-1H-thieno[3,4-d]imidazol
2-[4-(3,5-Bistrifluormethylbenzyloxy)-2-picolylsulfinyl]-1H-thieno[3,4-d]imidazol
2-[4-(2,4-Difluorphenoxy)-2-picolylsulfinyl]-1H-thieno-[3,4-d]imidazol
2-[4-(3-Trifluormethylphenoxy)-2-picolylsulfinyl]-1H-thieno[3,4-d]imidazol
2-[4-(4-Fluorphenoxy)-2-picolylsulfinyl]-1H-thieno[3,4-d] imidazol
2-[4-(4-Chlorphenoxy)-2-picolylsulfinyl]-1H-thieno[3,4-d] imidazol.

Alkyl und davon abgeleitete Reste wie beispielsweise Alkoxy oder Alkanoyl können geradkettig oder verzweigt sein.

Halogen steht für Fluor, Chlor, Brom oder Jod.

Geeignete $N^{im}$-Schutzgruppen $R^3$ sind z.B. im Zusammenhang mit substituierten Picolylsulfinylbenzimidazolen in EP-A-176 308 und EP-A2-221 041, für Thienoimidazolverbindungen in EP-A-234 485 beschrieben.

Bevorzugte $N^{im}$-Schutzgruppen sind solche, die in Gegenwart von Säuren, vorzugsweise in einem pH-Bereich von etwa 1 - 6 und/oder unter physiologischen Bedingungen abgespalten werden können.

Gegebenenfalls vorhandene chirale C- Atome können sowohl in der R- als auch in der S-Konfiguration vorkommen. In solchen Fällen liegen Verbindungen der Formel I in Form der reinen Enantiomeren oder als Stereoisomerengemisch (wie Enantiomerengemisch und Diastereomerengemisch) vor.

Als Salze kommen insbesondere Alkali- und Erdalkalisalze und Salze mit physiologisch verträglichen Aminen in Frage.

Die Erfindung betrifft weiterhin ein Verfahren zur Herstellung von Verbindungen der Formel I, das dadurch gekennzeichnet ist, daß man

a) Verbindungen der Formel II

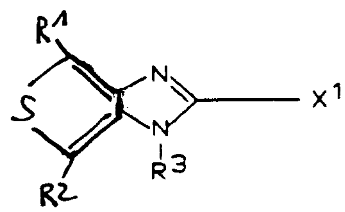

(II)

in welcher $R^1$, $R^2$ und $R^3$ wie oben definiert sind und

$X^1$

i. eine Abgangsgruppe oder
ii. -SH oder $-S^-M^+$ bedeutet,

umsetzt mit Verbindungen der Formel III

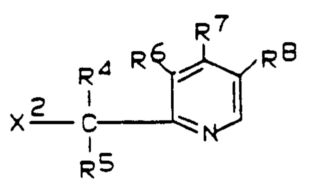

(III)

in welcher $R^4$, $R^5$, $R^6$, $R^7$ und $R^8$ wie oben definiert sind und

$X^2$ im oben genannten Fall i. -SH oder $-S^-M^+$ und im oben genannten Fall ii. vorzugsweise eine Abgangsgruppe oder OH bedeutet, oder

b) Verbindungen der Formel IV,

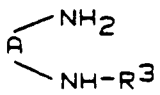

(IV)

in welcher A, $R^1$, $R^2$ und $R^3$ wie oben definiert sind, umsetzt mit Verbindungen der Formel V

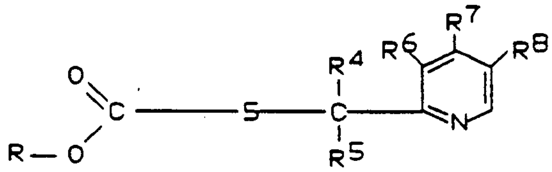

(V)

in welcher $R^4$, $R^5$, $R^6$, $R^7$ und $R^8$ wie oben definiert sind und R für eine veresternde Gruppe steht,

i. in Verbindungen der Formel I (eine) gegebenenfalls vorhandene -S-Gruppe(n) zu(r) -SO-Gruppe(n) oxidiert,

ii. Verbindungen der Formel I, worin $R^3$ für Wasserstoff steht, gewünschtenfalls acyliert, alkyliert oder aralkyliert,

iii. Verbindungen der Formel I, worin $R^3$ nicht Wasserstoff bedeutet, gewünschtenfalls verseift und

iv. Verbindungen der Formel I gewünschtenfalls in ihre physiologisch verträglichen Salze überführt,

wobei zwei oder mehr der Maßnahmen i.-iii. auch in einer anderen als der angegebenen Reihenfolge ausgeführt werden können.

$M^+$ steht für Kationen wie beispielsweise Alkali-, Erdalkali-, Ammonium- oder Alkylammonium-Ionen, insbesondere Natrium- oder Kalium-Ionen.

Setzt man gemäß der hier bevorzugten Verfahrensvariante (a) Verbindungen der Formel II mit Verbindungen der Formel III um, so steht $X^1$ oder $X^2$ für eine Abgangsgruppe, die nucleophil ablösbar ist, wie Cl, Br, J, $-O-SO_2-CH_3$, $-O-SO_2-CF_3$ oder $-O-SO_2-(C_6H_4-pCH_3)$.

Die Umsetzung einer Verbindung der Formel II mit einer Verbindung der Formel III oder deren Salzen erfolgt in einem inerten Lösungsmittel wie z.B. Wasser, Methylenchlorid, Methanol, Ethanol, Aceton, Essigsäureethylester, Toluol, Tetrahydrofuran, Acetonitril, Dimethylformamid, Dimethylsulfoxid oder Gemischen dieser Lösungsmittel zweckmäßigerweise in Gegenwart einer anorganischen oder organischen Base, wie z.B. Natrium- oder Kaliumhydroxid, -carbonat, -alkoxid, -hydrid, -amid, Ammoniak, Triethylamin, Tributylamin, Pyridin bei -20 bis +150 °C, vorzugsweise bei 0 - 80 °C.

Die Verbindungen der Formel II sind bekannte Verbindungen (siehe z. B. Gronowitz, "The Chemistry of Heterocyclic Compounds", Band 44, "Thiophene and its Derivatives", Parts 1 - 3, New York 1985-6) oder können in Analogie zu bekannten Verfahren hergestellt werden, z.B. durch Ringschluß entsprechend substituierter 3,4-Diaminothiophene der oben definierten Formel IV mit entsprechenden Schwefelverbindungen wie Schwefelkohlenstoff (z.B. DE-A-31 32 167).

Die hierfür benötigten 3,4- Diaminothiophene sind entweder literaturbekannt oder können in Analogie zu bekannten Verfahren hergestellt werden. Sie werden z.B. durch Reduktion entsprechend substituierter Aminonitrothiophene erhalten.

In den bei Verfahrensvariante (b) eingesetzten Estern der Formel V steht R für eine veresternde Gruppe, vorzugsweise $(C_1-C_6)$-Alkyl oder Benzyl.

Die Umsetzung einer Verbindung der Formel IV mit einer Verbindung der Formel V gemäß Verfahrensvariante (b) erfolgt analog der in Preston et al., Benzimidazoles and Congeneric Tricyclic Compounds, Part 1, New York, Seiten 10-13 beschriebenen Verfahrensweisen.

Die so erhaltenen Verbindungen der Formel I können, falls $R^3$ Wasserstoff bedeutet, in physiologisch verträgliche Salze umgewandelt werden.

In gleicher Weise lassen sich auch -S-Gruppen in den Substituenten $R^1$, $R^2$ und $R^7$ oxidieren.

Diese Reaktion erfolgt in einem geeigneten, inerten Lösungsmittel wie z:B. Methylenchlorid, Chloroform, Tetrachlorkohlenstoff, 1,2-Dichlorethan, Toluol, Essigsäureethylester, Essigsäure, Trifluoressigsäure, Wasser, Methanol, Ethanol oder Gemischen derselben bei -20 °C bis +150 °C vorzugsweise bei -10 °C bis +40 °C.

Als Oxidationsmittel kommen z.B. in Betracht: Wasserstoffperoxid, Persäuren und Perester wie Peressigsäure, Trifluorperessigsäure, Monoperphthalsäure, m-Chlorperbenzoesäure und deren Ester, Ozon, Distickstofftetroxid, Jodosobenzol, N-Chlorsuccinimid, 1-Chlorbenzotriazol, Natriumhypochlorit, Kaliumperoxodisulfat, t-Butylhypochlorit, Tetrabutylammoniumperjodat oder -permanganat, Natrium-meta-perjodat, Selen- oder Mangandioxid, Cerammonnitrat, Chromsäure, Chlor, Brom, Diazabicyclo-[2.2.2]octan-Bromkomplex, Dioxandibromid, Pyridiniumperbromid, Sulfurylchlorid, 2-Arylsulfonyl-3-aryloxaziridine, Titantetraisopropylat/tert. Butylhydroperoxid (gegebenenfalls unter Zusatz von Dialkylestern der (D)- bzw. (L)-Weinsäure und einer definierten Menge Wasser).

Ebenso können isolierte, ggf. immobilisierte oxidierende Enzyme oder Mikroorganismen als Oxidationsmittel Anwendung finden.

Die Oxidationsmittel werden in äquimolaren Mengen, ggfs. auch in einem geringen Überschuß von 5 - 10 Mol-% bei der Oxidation zu -SO-eingesetzt.

Die Erfindung betrifft weiterhin neue Zwischenprodukte der Formel III. Sie können nach dem Fachmann bekannten Methoden, wie sie beispielsweise in "The Chemistry of Heterocyclic Compounds - Pyridine and its Derivatives", Pts. 2 and 3, E. Klingsberg Ed. Interscience Publishers, 1962, beschrieben sind, hergestellt werden.

Neben der Synthese der Verbindungen der Formel III, wobei $X^2$ eine Abgangsgruppe bedeutet, aus Verbindungen der Formel III, worin $X^2$ eine Hydroxygruppe bedeutet, können die Verbindungen der Formel III, in der $X^2$ Chlor oder Brom bedeuten, z. B. durch Halogenierung der entsprechenden 2-Picoline mit N-Bromsuccinimid, Trichlorisocyanursäure (Chem. Ber. 120, 649-651 (1987)) oder anderen N-Halogenamiden wie N-Chlorphthalimid hergestellt werden.

Ohne die Erfindung auf die folgenden Beispiele zu begrenzen, sind nachstehend einige Herstellungsverfahren für Verbindungen der Formel III, in denen $X^2$ Hydroxy bedeutet, beschrieben. Deren Umwandlung in Verbindungen der Formel III, in denen $X^2$ eine Abgangsgruppe bedeutet, erfolgt nach Standardmethoden.

Verbindungen der Formel III, in denen $X^2$ eine Hydroxygruppe, $R^6$ und $R^8$ gleich oder verschieden Wasserstoff oder Methyl, und $R^7$ einen ein- oder mehrfach substituierten ($C_6$-$C_{12}$)-Aryloxy- oder ($C_7$-$C_{11}$)-Aralkyloxyrest bedeutet, werden aus Nitroverbindungen der Formel VI oder Chlorverbindungen der Formel VII erhalten.

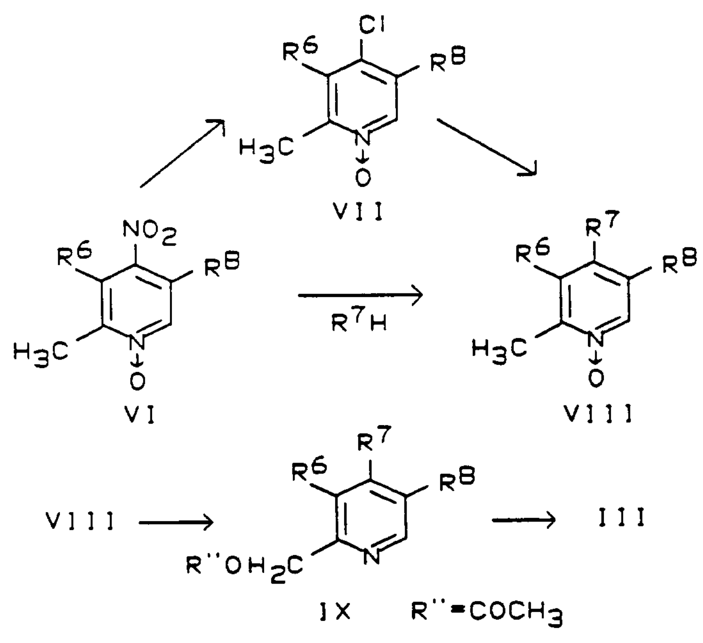


Eine Verbindung der Formel VI oder VII wird in einem inerten Lösungsmittel wie beispielsweise Tetrahydrofuran, Dioxan, Acetonitril, Aceton, Methylethylketon, Dimethylsulfoxid, Dimethylformamid, Dimethylacetamid, Hexamethylphosphorsäuretriamid oder 1,3-Dimethyl-3,4,5,6-tetrahydro-2-(1H)-pyrimidinon, in Gegenwart einer organischen oder anorganischen Base wie beispielsweise Natriumhydroxid, -hydrogencarbonat, -carbonat, -hydrid, -propylat, Lithiumcarbonat, Kaliumhydroxid, -carbonat, -hydrid, -hydrogencarbonat oder Kalium-tert.butylat mit einem Alkohol der Formel $R^7H$ zwischen 0 °C und dem Siedepunkt des Lösungsmittels zur Reaktion gebracht.

Die Herstellung der Verbindungen der Formel III aus Verbindungen der Formel VII, die durch Einwirkung von chlorierenden Agenzien wie z. B. Acetylchlorid, Thionylchlorid und Phosphoroxychlorid auf Verbindungen der Formel VI erhalten werden, kann gegenüber der Reaktion mit Verbindungen der Formel VI von Vorteil sein.

Auch 4-Fluor-2-picolin, (J. Prakt. Chem. 9, 164 - 172 (1959)) und das zu VII analoge 4-Fluor-2-picolin-N-oxid können Verwendung finden.

Ohne die Erfindung auf die im folgenden genannten Beispiele zu begrenzen, werden z. B. 4-Fluor-, 4-Chlor-, 4-Trifluormethyl-, 2,4-Difluor-, 3,5-Difluor-, 3,5-Dichlor-, 3,5-Bistrifluormethyl- und Pentafluorbenzylalkohol, 4-Fluor-, 4-Chlor-, 3,4-Dichlor-, 3-Trifluormethyl-, 4-Trifluormethyl-, 2,4-Difluorphenol und Pentafluorphenol als Alkohole eingesetzt.

Die so erhaltenen Verbindungen der Formel VIII werden mit Trifluoracetanhydrid bei 0 °C oder mit Acetanhydrid, evtl. in Gegenwart einer Säure, wie z. B. Eisessig bei 80 - 120 °C zu den Acetaten der Formel IX umgesetzt.

Deren alkalische Hydrolyse mit Alkalihydroxiden oder -carbonaten in Methanol, Ethanol, Wasser oder ähnliches, führt zu den Verbindungen der Formel III, in denen $X^2$ Hydroxy bedeutet.

Verbindungen der Formel III, in denen $X^2$ eine Hydroxygruppe, $R^6$ ein Alkoxyrest, $R^a$ $(C_1\text{-}C_6)$-Alkyl oder $(C_7\text{-}C_{10})$-Aralkyl und $R^8$ Wasserstoff bedeutet, können nach folgendem Verfahren hergestellt werden.

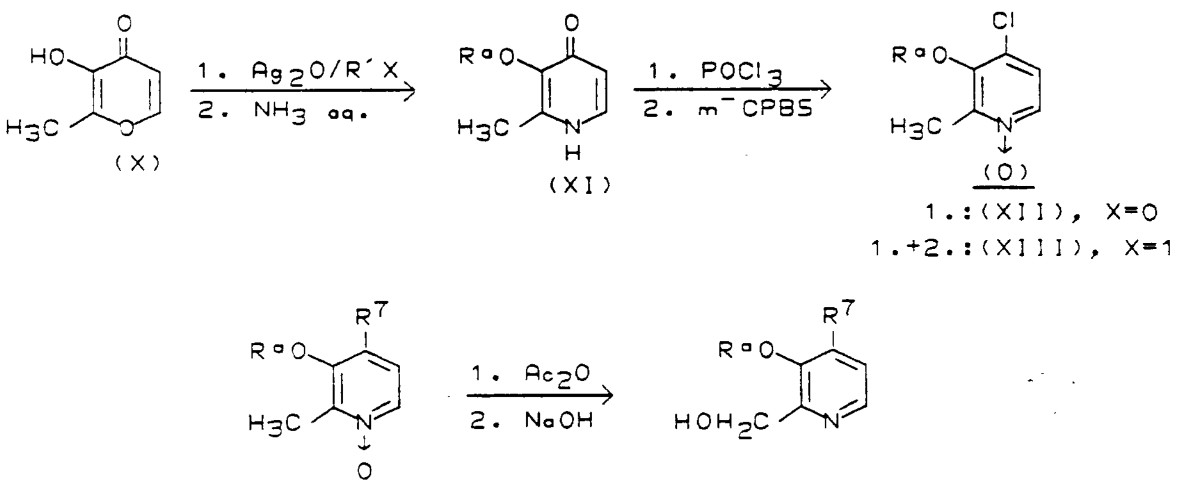

(X) (XI) (XIV) (III)

Maltol X reagiert beispielsweise mit einem Halogenid $R^aX$ in Gegenwart von Silberoxid oder mit Fluoralkyltriflaten zu 3-Alkoxy-pyran-4-onen, die mit wäßrigem Ammoniak zu 2-Methyl-3-alkoxy-4-pyridonen der Formel XI umgesetzt werden (J. Org. Chem. 29, 776 (1964)).

Mit einem halogenierenden Agenz, z. B. $POCl_3$, werden die Verbindungen der Formel XI in 2-Methyl-3-alkoxy-4-chlorpyridine überführt, aus denen mit einem Alkohol $R^7H$ in Gegenwart einer Base 4-Alkoxyderivate erhalten werden.

Vorteilhaft ist die Reaktion der analogen N-Oxide XIII mit Alkoholaten zu Verbindungen der Formel XIV.

Die zur Anwendung kommenden substituierten Benzylalkohole und Phenole sind vorstehend genannt.

Verbindungen der Formel XI können in Gegenwart von Silberoxid auch direkt zu Verbindungen der Formel XV umgesetzt werden.

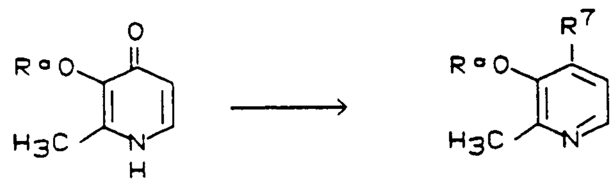
XI XV

Die Erfindung betrifft weiterhin Verbindungen der Formel III, die dadurch erhalten werden, daß in analoger Art und Weise anstelle von Maltol X das isomere 5-Hydroxy-2-methyl-4-pyranon der Formel XVII, das z. B. wie in J. Chem. Soc. 1956, 2558, beschrieben, aus Kojic Säure XVI hergestellt wird, eingesetzt werden.

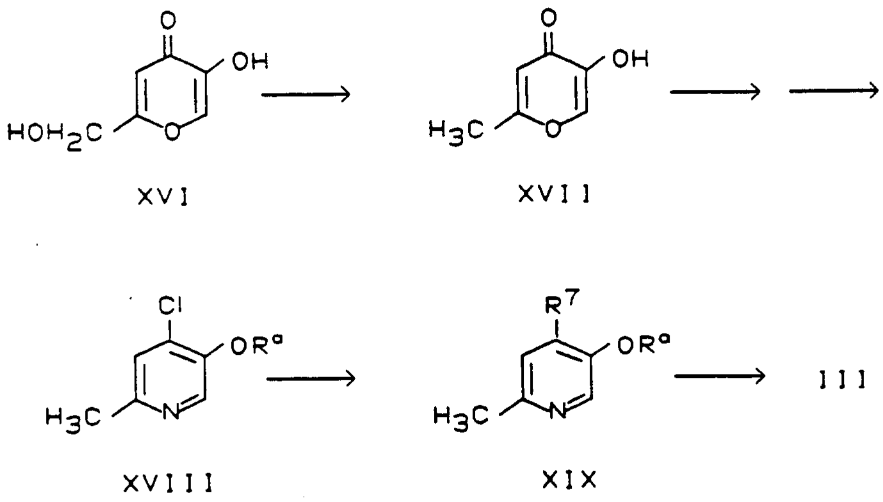

XVI XVII XVIII XIX III

Verfahren zur Herstellung von 3,4-Dialkoxy-2-picolinen und 4,5-Dialkoxy-2-picolinen sind auch in den EP-A-166 287 und EP-A-208 452 beschrieben.

Die neuen Verbindungen der Formel I und ihre Salze besitzen wertvolle pharmakologische Eigenschaften.

Sie hemmen deutlich die Magensäuresekretion und weisen darüber hinaus eine ausgezeichnete Magen- und Darmschutzwirkung auf.

Unter "Magen- und Darmschutz" wird in diesem Zusammenhang die Verhütung und Behandlung gastrointestinaler Krankheiten, insbesondere gastrointestinaler entzündlicher Krankheiten und Läsionen (wie z.B. Ulcus ventriculi, Ulcus duodeni, Gastritis, hyperazider oder medikamentös bedingter Reizmagen) verstanden, die beispielsweise durch Mikroorganismen, Bakterientoxine, Medikamente (z.B. Antiphlogistika und Antirheumatika), Chemikalien (z.B. Ethanol), Magensäure oder Streßsituationen verursacht werden können.

Aufgrund ihrer ausgezeichneten Eigenschaften sind die substituierten Thienoimidazole der Formel I und ihre pharmakologisch verträglichen Salze für den Einsatz in der Human- und Veterinärmedizin hervorragend geeignet, wobei sie insbesondere zur Behandlung und Prophylaxe von Krankheiten des Magens und Darms und solcher Krankheiten, die auf einer überhöhten Magensäuresekretion beruhen, verwendet werden.

Es wurde gefunden, daß auch die Colon-$H^+/K^+$-ATPase (vgl. [Gustin, Goodman, J. Biol. Chem. 256 - [1981] 10651-10656 in vitro stark durch Verbindungen gehemmt wird, die beim Behandeln der erfindungsgemäßen Verbindungen der Formel I mit Säure (z.B. mit Natriumacetat/HCl-Puffer mit einem pH-Wert von etwa 4-5,5) entstehen. Solche Umwandlungsprodukte können sich auch in vivo bei der Passage der Verbindungen der Formel I durch den Magen-Darm-Trakt bilden. In welchem Umfang sie gebildet werden, hängt vom Substitutionsmuster und vom pH ab.

Der Colon-$H^+/K^+$-ATPase wird ein entscheidender Einfluß auf das Elektrolytgleichegewicht an der Darmschleimhaut zugeschrieben. Colon-$H^+/K^+$-ATPase-Hemmer, wie die oben genannten, können daher in dieses Gleichgewicht eingreifen und zur Behandlung von Krankheiten mit gestörtem Elektrolytgleichgewicht dienen.

Die Erfindung betrifft daher auch die Verwendung von Verbindungen der Formel I bzw. deren Säure-Umwandlungsprodukte bei der Behandlung von Durchfallerkrankungen. Beispiele solcher Krankheiten sind entzündliche Darmerkrankungen, wie Cholera, Paratyphus, Reisediarrhoe oder andere Formen der sekretorischen Diarrhoe aber auch colitis ulcerosa, Morbus Crohn und regionale Enteritis.

Die Erfindung betrifft weiterhin Umwandlungsprodukte, die beim Behandeln von Verbindungen der Formel I mit Säure gebildet werden.

Die Erfindung betrifft daher weiter die erfindungsgemäßen Verbindungen der Formel I zur Anwendung bei der Behandlung und Prophylaxe der vorstehend genannten Krankheiten.

Ebenso umfaßt die Erfindung die Verwendung der erfindungsgemäßen Verbindungen bei der Herstellung von Arzneimitteln, die zur Behandlung und Prophylaxe der vorstehend genannten Krankheiten eingesetzt werden.

Ein weiterer Gegenstand der Erfindung sind Arzneimittel, die ein oder mehrere Verbindungen der allgemeinen Formel I und/oder ihre pharmakologisch verträglichen Salze enthalten sowie Einschlußverbindungen aus Verbindungen der Formel I und Cyclodextrin, vorzugsweise $\beta$-Cyclodextrin.

Die Arzneimittel werden nach an sich bekannten, dem Fachmann geläufigen Verfahren hergestellt. Als Arzneimittel werden die erfindungsgemäßen pharmakologisch wirksamen Verbindungen (= Wirkstoffe) entweder als solche, oder vorzugsweise in Kombination mit geeigneten pharmazeutischen Hilfsstoffen in Form von Tabletten, Dragees, Kapseln, Suppositorien, Emulsionen, Suspensionen oder Lösungen eingesetzt, wobei der Wirkstoffgehalt vorteilhafterweise zwischen 0,1 und 96 % beträgt.

Welche Hilfsstoffe für die gewünschte Arzneimittelformulierungen geeignet sind, ist dem Fachmann aufgrund seines Wissens geläufig. Neben Lösemitteln, Gelbildern, Suppositoriengrundlagen, Tabletten-Hilfsstoffen und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Die Wirkstoffe können oral oder parenteral appliziert werden, wobei die orale Applikation bevorzugt ist.

Im allgemeinen hat es sich in der Humanmedizin als vorteilhaft erwiesen, den oder die Wirkstoffe bei oraler Gabe in einer Tagesdosis von etwa 0,01 bis etwa 20 mg/kg Körpergewicht, gegebenenfalls in Form mehrerer, vorzugsweise 1 bis 4 Einzelgaben zur Erzielung des gewünschten Ergebnisses zu verabreichen. Bei der parenteralen Applikation können ähnliche bzw. (insbesondere bei der intravenösen Verabreichung der Wirkstoffe) in der Regel niedrigere Dosierungen zur Anwendung kommen. Die Festlegung der jeweils erforderlichen optimalen Dosierung und Applikationsart der Wirkstoffe kann durch jeden Fachmann aufgrund seines Fachwissens leicht erfolgen.

Sollen die erfindungsgemäßen Verbindungen und/oder ihre Salze zur Behandlung der obengenannten Krankheiten eingesetzt werden, so können die pharmazeutischen Zubereitungen auch einen oder mehrere pharmakologisch aktive Bestandteile anderer Arzneimittelgruppen, wie Antibiotika, beispielsweise Ofloxazin, Antacida, beispielsweise Aluminiumhydroxid, Magnesiumaluminat, Sucralfat, Bi-Salze, Tranquilizer, wie Benzodiazepine, beispielsweise Diazepam; Spasmolytika, wie z.B. Bietamiverin, Camylofin; Anticholinergica, wie z.B. Pirenzepin, Telezepin, Oxyphencyclimin, Phencarbamid; Lokalanaesthetika, wie z. B. Tetracain, Procain; gegebenenfalls auch Gastrinantagonisten, Fermente, Vitamine oder Aminosäuren enthalten.

Für eine orale Anwendungsform werden die aktiven Verbindungen mit den dafür üblichen Zusatzstoffen wie Trägerstoffen, Stabilisatoren oder inerten Verdünnungsmitteln vermischt und durch übliche Methoden in geeignete Darreichungsformen gebracht, wie Tabletten, Dragees, Steckkapseln, wäßrige, alkoholische oder ölige Suspensionen oder wäßrige alkoholische oder ölige Lösungen. Als inerte Träger können z.B. Gummi arabicum, Magnesia, Magnesiumcarbonat, Milchzucker, Glucose oder Stärke, insbesondere Maisstärke verwendet werden. Dabei kann die Zubereitung sowohl als Trocken- oder Feuchtgranulat erfolgen. Als ölige Trägerstoffe oder Lösungsmittel kommen beispielsweise pflanzliche und tierische Öle in Betracht, wie Sonnenblumenöl oder Lebertran.

Zur subkutanen oder intravenösen Applikation werden die aktiven Verbindungen oder deren physiologisch verträgliche Salze, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel für die neuen aktiven Verbindungen und die entsprechenden physiologisch verträglichen Salze kommen z.B. in Frage: Wasser, physiologische Kochsalzlösungen oder Alkohole, z.B. Ethanol, Propanol oder Glycerin, daneben auch Zuckerlösungen wie Glucose- oder Mannitlösungen, oder auch eine Mischung aus den verschiedenen genannten Lösungsmitteln.

Die nachfolgenden Beispiele sollen die erfindungsgemäßen Verfahrensweisen erläutern, ohne die Erfindung auf die hier stellvertretend genannten Substanzen zu beschränken.

Die angegebenen Schmelz- und Zersetzungspunkte sind nicht korrigiert oder standardisiert.

**Beispiel 1**

4-Chlor-2-picolin-N-oxid

75 ml Acetylchlorid werden bei 0 °C portionsweise mit 15,4 g (0,1 Mol) 4-Nitro-2-picolin-N-oxid versetzt. Beim Erwärmen auf Raumtemperatur entsteht eine klare Lösung, die unter Rühren auf Eis getropft wird. Nach Hinzufügen von $K_2CO_3$ wird mehrfach mit Dichlormethan und Ethylacetat extrahiert. Nach Eindampfen wird das Produkt chromatographisch an Kieselgel gereinigt. Das erhaltene Öl kristallisiert beim Stehen, Fp. 37 °C.

**Beispiel 2**

4-(4-Trifluormethyl-benzyloxy)-2-picolin-N-oxid

25 ml (183 mMol) 4-Trifluormethylbenzylalkohol werden bei 25 °C unter $N_2$-Atmosphäre mit 11,2 g (100 mMol) Kalium-tert. Butylat versetzt. Anschließend tropft man 7,2 g (50 mMol) 4-Chlor-2-picolin-N-oxid hinzu, versetzt dann mit 10 ml tert. Butanol, rührt 1 h bei 25 °C und 30 min bei 75 - 80 °C. Sodann wird mit Wasser versetzt und dreimal mit Dichlormethan extrahiert, getrocknet, eingeengt und der Rückstand mit Dichlormethan/Methanol an Kieselgel chromatographiert. Aus entsprechenden Fraktionen erhält man das Produkt, Fp. 113 - 115 °C.

**Beispiel 3**

4-(4-Trifluormethyl-benzyloxy)-2-hydroxymethylpyridin

3,4 g (12 mMol) der Titelverbindung aus Beispiel 2 werden in 5 ml Eisessig auf 80 °C erwärmt und unter Rühren bei dieser Temperatur tropfenweise mit 10 ml Acetanhydrid versetzt. Dann erwärmt man 2 h auf 110 - 120 °C, läßt auf 80 °C abkühlen, gibt 10 ml Methanol zu, erhitzt weitere 15 min zum Rückfluß und engt anschließend ein. Der Rückstand wird in 10 ml Methanol aufgenommen und bei 10 °C rasch in 50 ml 2N methanolische NaOH eingetropft. Die Lösung wird über Kohle geklärt, eingeengt, der Rückstand mit 50 ml behandelt, dreimal mit 50 ml Dichlormethan extrahiert, die organische Phase getrocknet und eingeengt. Der auskristallisierte Rückstand wird mit einem Petrolether/Diisopropylether-Gemisch (1:1) verrieben, das Produkt abgesaugt, mit wenig Diisopropylether gewaschen und im Vakuum getrocknet, Fp. 96 - 98 °C.

**Beispiel 4**

4-(4-Trifluormethyl-benzyloxy)-2-chlormethylpyridin

2,2 g (7,8 mMol) der Titelverbindung aus Beispiel 3 werden in 50 ml wasserfreiem Dichlormethan gelöst und bei -10 °C tropfenweise mit einer Lösung von 2 ml Thionylchlorid in 6 ml Dichlormethan versetzt. Man erwärmt auf Raumtemperatur, rührt noch 30 min, engt ein. Der kristalline Rückstand wird mit Diisopropylether zur Kristallisation gebracht und an der Ölpumpe getrocknet, Fp. 133 - 135 °C.

**Beispiel 5**

2-[4-(4-Trifluormethyl-benzyloxy)-2-picolylmercapto]-1H-thieno[3,4-d]imidazol-dihydrochlorid

1,35 g (4 mMol) der Titelverbindung aus Beispiel 4 werden in 50 ml Ethanol bei 25 °C mit 0,625 g (4 mMol) 2-Mercapto-thieno[3,4-d]imidazol versetzt und 1,5 h bei 60 °C gerührt. Die Reaktionsmischung wird im Vakuum eingeengt, der Rückstand mit Aceton behandelt, abgesaugt, mit Aceton gewaschen und im Vakuum getrocknet, Fp. 180 - 182 °C.

**Beispiel 6**

2-[4-(4-Trifluormethyl-benzyloxy)-2-picolylmercapto]-1H-thieno[3,4-d]imidazol

1,5 g (3,0 mMol) des Dihydrochlorid aus Beispiel 5 werden unter Eiskühlung und Stickstoffatmosphäre in 25 ml Methanol suspendiert und tropfenweise mit 1,7 ml (ca. 12 mMol) Triethylamin versetzt, wobei sich eine klare Lösung bildet, die anschließend über Aktivkohle geklärt und eingeengt wird. Beim Behandeln mit Wasser kristallisiert der Rückstand, wird mit Wasser gewaschen und im Vakuum getrocknet, Fp. 141 - 143 °C unter Zersetzung.

**Beispiel 7**

2-[4-(4-Trifluormethyl-benzyloxy)-2-picolylsulfinyl]-1H-thieno[3,4-d]imidazol

Zu 1,0 g (2,37 mMol) der Titelverbindung aus Beispiel 6 im Zweiphasengemisch aus 200 ml Dichlormethan und 100 ml wäßriger $KH_2PO_4$/$Na_2HPO_4$-Pufferlösung (pH = 7,5) wird unter Rühren bei 0 bis

5°C eine Lösung von 0,5 g (2,5 mMol, 85 %) m-Chlorperbenzoesäure in 10 ml Dichlormethan zugetropft. Die organische Phase wird abgetrennt, mit gesättigter wäßriger $NaHCO_3$-Lösung ausgeschüttelt, über $MgSO_4$ getrocknet, über Aktivkohle geklärt und eingeengt. Der kristalline Eindampfrückstand wird mit Ethylacetat behandelt, abgesaugt und zweimal mit wenig Ethylacetat gewaschen. Das farblos kristalline Produkt wird im Vakuum getrocknet, Fp. 149°C unter Zersetzung.

**Beispiel 8**

4-(2,4-Difluorphenoxy)-2-picolin-N-oxid

Zu einer Mischung aus 4,6 g (35 mMol) 2,4-Difluorphenol in 25 ml N,N-Dimethylacetamid und 4,9 g (35 mMol) fein gepulvertem Kaliumcarbonat gibt man 4,3 g (30 mMol) 4-Chlor-2-picolin-N-oxid und erwärmt unter Rühren 3 h auf 140°C. Nach dem Abkühlen wird die Reaktionsmischung in 250 ml Wasser gegossen, dreimal mit Dichlormethan extrahiert, die organische Phase über $MgSO_4$ getrocknet und eingeengt. Der Rückstand wird in Dichlormethan aufgenommen und mit gesättigter wäßriger NaCl-Lösung ausgeschüttelt. Nach dem Trocknen und Befreien vom Lösungsmittel wird mit Diisopropylether zur Kristallisation gebracht und mit Ethylacetat gewaschen. Farblose Kristalle, Fp. 112 - 124°C.

Die folgenden Beispiele 9 bis 72 wurden analog den Beispielen 1 bis 8 sowie den aufgeführten Syntheseschemata hergestellt:

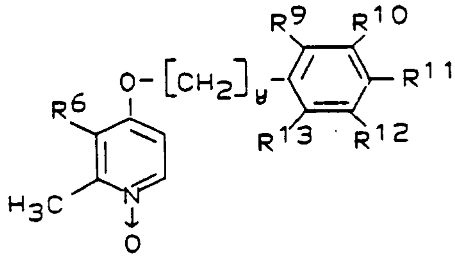

| Bsp.Nr. | y | $R^6$ | $R^9$ | $R^{10}$ | $R^{11}$ | $R^{12}$ | $R^{13}$ | Fp.[°C] |
|---|---|---|---|---|---|---|---|---|
| 9 | 1 | H | H | H | F | H | H | 155-157 |
| 10 | 1 | H | H | $CF_3$ | H | $CF_3$ | H | 162-164 |
| 11 | 1 | H | F | H | F | H | H | 145-148 |
| 12 | 1 | H | H | F | H | F | H | * |
| 13 | 1 | H | H | F | F | H | H | 170-172 |
| 14 | 1 | H | H | H | Cl | H | H | 153-155 |
| 15 | 1 | H | H | Cl | H | Cl | H | 163-165 |
| 16 | 1 | $OCH_3$ | H | H | F | H | H | 78-80 |
| 17 | 1 | $OCH_3$ | H | H | $CF_3$ | H | H | 146-148 |
| 18 | 1 | $OCH_3$ | H | $CF_3$ | H | $CF_3$ | H | 164-167 |
| 19 | 1 | $OCH_3$ | H | F | F | H | H | * |
| 20 | 0 | H | H | $CF_3$ | H | H | H | Öl * |

* ohne zu reinigen, weiter umgesetzt

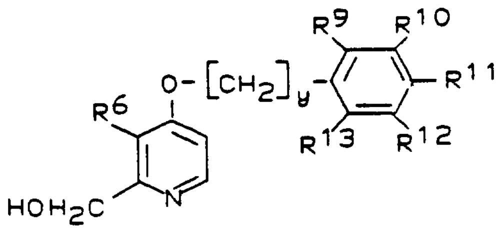

| Bsp.Nr. | y | $R^6$ | $R^9$ | $R^{10}$ | $R^{11}$ | $R^{12}$ | $R^{13}$ | Fp.[°C] |
|---|---|---|---|---|---|---|---|---|
| 21 | 1 | H | H | H | F | H | H | 74-76 |
| 22 | 1 | H | H | $CF_3$ | H | $CF_3$ | H | 113-115 |
| 23 | 1 | H | F | H | F | H | H | 56-60 |
| 24 | 1 | H | H | F | H | F | H | Öl * |
| 25 | 1 | H | H | F | F | H | H | Öl * |
| 26 | 1 | H | H | H | Cl | H | H | 79-82 |
| 27 | 1 | H | H | Cl | H | Cl | H | 114-115 |
| 28 | 1 | $OCH_3$ | H | H | F | H | H | 106-107 |
| 29 | 1 | $OCH_3$ | H | H | $CF_3$ | H | H | 100-101 |
| 30 | 1 | $OCH_3$ | H | $CF_3$ | H | $CF_3$ | H | 88-90 |
| 31 | 1 | $OCH_3$ | H | F | F | H | H | 93-95 |
| 32 | 0 | H | H | $CF_3$ | H | H | H | Öl * |
| 33 | 0 | H | F | H | F | H | H | 62-64 |

* weiter umgesetzt

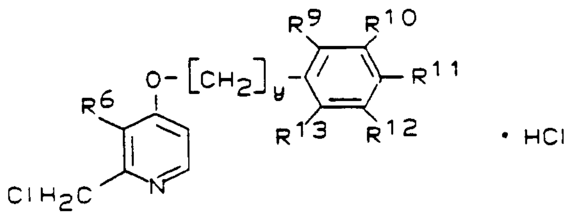

| Bsp.Nr. | y | $R^6$ | $R^9$ | $R^{10}$ | $R^{11}$ | $R^{12}$ | $R^{13}$ | Fp.[°C] |
|---|---|---|---|---|---|---|---|---|
| 34 | 1 | H | H | H | F | H | H | 142-143 |
| 35 | 1 | H | H | $CF_3$ | H | $CF_3$ | H | 167-169 |
| 36 | 1 | H | F | H | F | H | H | 143-144 |
| 37 | 1 | H | H | F | H | F | H | hygroskopisch |
| 38 | 1 | H | H | F | F | H | H | 136-138 |
| 39 | 1 | H | H | H | Cl | H | H | 154-156 |
| 40 | 1 | H | H | Cl | H | Cl | H | 159-160 |
| 41 | 1 | $OCH_3$ | H | H | F | H | H | 133-135 |
| 42 | 1 | $OCH_3$ | H | H | $CF_3$ | H | H | 155-156 |
| 43 | 1 | $OCH_3$ | H | $CF_3$ | H | $CF_3$ | H | 151-152 |
| 44 | 1 | $OCH_3$ | H | F | F | H | H | hygroskopisch |
| 45 | 0 | H | H | $CF_3$ | H | H | H | Harz * |
| 46 | 0 | H | F | H | F | H | H | 97-100 |

* weiter umgesetzt

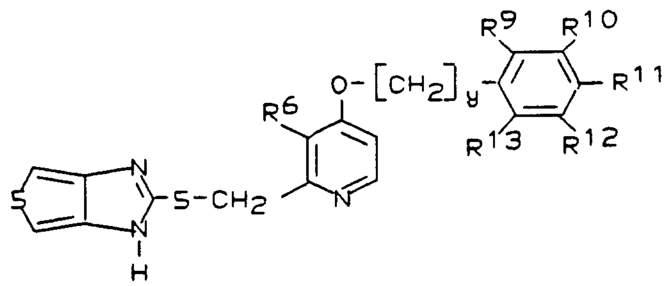

| Bsp.Nr. | y | $R^6$ | $R^9$ | $R^{10}$ | $R^{11}$ | $R^{12}$ | $R^{13}$ | Fp.[°C] |
|---|---|---|---|---|---|---|---|---|
| 47 | 1 | H | H | H | F | H | H | 141 |
| 48 | 1 | H | H | $CF_3$ | H | $CF_3$ | H | 187 |
| 49 | 1 | H | F | H | F | H | H | 160-161 |
| 50 | 1 | H | H | F | H | F | H | 142-143 |
| 51 | 1 | H | H | F | F | H | H | 143-145 |
| 52 | 1 | H | H | H | Cl | H | H | 158-159 |
| 53 | 1 | H | H | Cl | H | Cl | H | 178-179 |
| 54 | 1 | $OCH_3$ | H | H | F | H | H | 147-149 |
| 55 | 1 | $OCH_3$ | H | H | $CF_3$ | H | H | 138-139 |
| 56 | 1 | $OCH_3$ | H | $CF_3$ | H | $CF_3$ | H | 135-137 |
| 57 | 1 | $OCH_3$ | H | F | F | H | H | 150 |
| 58 | 0 | H | H | $CF_3$ | H | H | H | 154-155 |
| 59 | 0 | H | F | H | F | H | H | 129-131 |

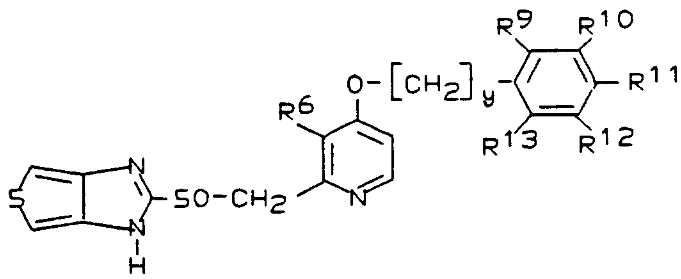

| Bsp.Nr. | y | $R^6$ | $R^9$ | $R^{10}$ | $R^{11}$ | $R^{12}$ | $R^{13}$ | Fp. [°C] unter Zers. |
|---|---|---|---|---|---|---|---|---|
| 60 | 1 | H | H | H | F | H | H | 145 |
| 61 | 1 | H | H | $CF_3$ | H | $CF_3$ | H | 135-136 |
| 62 | 1 | H | F | H | F | H | H | 117-118 |
| 63 | 1 | H | H | F | H | F | H | 159-161 |
| 64 | 1 | H | H | F | F | H | H | 151 |
| 65 | 1 | H | H | H | Cl | H | H | 144-145 |
| 66 | 1 | H | H | Cl | H | Cl | H | 102-104 |
| 67 | 1 | $OCH_3$ | H | H | F | H | H | 119-121 |
| 68 | 1 | $OCH_3$ | H | H | $CF_3$ | H | H | 148-149 |
| 69 | 1 | $OCH_3$ | H | $CF_3$ | H | $CF_3$ | H | 157-158 |
| 70 | 1 | $OCH_3$ | H | F | F | H | H | 120 |
| 71 | 0 | H | H | $CF_3$ | H | H | H | 119-121 |
| 72 | 0 | H | F | H | F | H | H | 122-124 |

Falls nicht explicit beschrieben, werden die nachfolgenden Beispiele analog den Beispielen 2 - 8 erhalten:

**Beispiel 73**

4-(4-Fluorphenoxy)-2-picolin-N-oxid

6,2 g (55 mMol) 4-Fluorphenol werden in 75 ml N,N-Dimethylacetamid gelöst und mit 20,7 g (150 mMol) fein gepulvertem Kaliumcarbonat versetzt. Zu dieser Suspension gibt man 7,7 g (50 mMol) 4-Nitro-2-picolin-N-oxid und erwärmt 3 Stunden auf 80° C. Anschließend werden weitere 7 g (50 mMol) Kaliumcarbonat zugegeben und eine Stunde bei 100° C gerührt. Nach dem Abkühlen wird in Vakuum vom N,N-Dimethylacetamid befreit, der Rückstand in Wasser aufgenommen, mehrfach mit Dichlormethan extrahiert, über $MgSO_4$ getrocknet, eingeengt und der Rückstand mit Diisopropylether zur Kristallisation gebracht, Fp. 122 - 124° C.

**Beispiel 74**

4-(4-Fluorphenoxy)-2-hydroxy-methylpyridin, Fp. 75 - 77° C (aus Diisopropylether)

**Beispiel 75**

4-(4-Fluorphenoxy)-2-chlormethylpyridin-hydrochlorid, öliges Rohprodukt, in Beispiel 76 weiter umgesetzt.

**Beispiel 76**

2-[4-(4-Fluorphenoxy)-2-picolylmercapto]-14-thieno[3,4-d]imidazol

1,60g(10 mMol) 2-Mercapto-thieno[3,4-d]imidazol werden in 100 ml Methanol suspendiert und mit 7 ml 5,5 m Natriummethylat-Lösung versetzt. Zu der entstandenen klaren Lösung tropft man bei Raumtemperatur eine Lösung von 3,0 g (10 mMol) der Titelverbindung aus Beispiel 75 in 20 ml Methanol und erhitzt 1 Stunde unter Rückfluß. Die Lösung wird eingeengt, der Rückstand mit Wasser versetzt und mit Dichlormethan extrahiert. Die organische Phase wird über $MgSO_4$ getrocknet, eingeengt und der Rückstand mit Diethylether zur Kristallisation gebracht. Das hellbraune Rohprodukt wird aus Ethylacetat/Aktivkohle umkristallisiert, Fp. 157 - 159° C.

**Beispiel 77**

2-[4-(4-Fluorphenoxy)-2-picolylsulfinyl]-1H-thieno[3,4-d]imidazol, Fp. 146° C [Zers.] (aus Ethylacetat).

**Beispiel 78**

4-(4-Chlorphenoxy)-2-picolin-N-oxid, Fp. 81 - 83° C (aus Diisopropylether), Herstellung analog Beispiel 73.

**Beispiel 79**

4-(4-Chlorphenoxy)-2-hydroxymethyl-pyridin, Fp. 64 - 65° C (aus Diisopropylether)

**Beispiel 80**

4-(4-Chlorphenoxy)-2-chlormethyl-pyridin-hydrochlorid, Fp. 156 - 157° C (aus Ethylacetat)

**Beispiel 81**

2-[4-(4-Chlorphenoxy)-2-picolyl-mercapto]-1H-thieno[3,4-d]imidazol, Fp. 150 - 151° C [Zers.] (aus Ethylacetat/Diethylether)

**Beispiel 82**

2-[4-(4-Chlorphenoxy)-2-picolyl-sulfinyl]-1H-thieno[3,4-d]imidazol, Fp. 140 - 141° C [Zers.] (aus Ethylacetat)

**Beispiel 83**

4-(3,5-Bistrifluormethylphenoxy)-2-picolin-N-oxid

a) Zu 3,45 g(15mMol) 3,5-Bistrifluormethylphenol, gelöst in 10 ml tert. Butanol, werden bei 20° C unter kräftigem Rühren und Stickstoffatmosphäre portionsweise 1,84 g (16,5 mMol) Kalium-tert. Butylat eingetragen. Anschließend wird tert. Butanol abdestilliert, der Rückstand in 10 ml N,N-Dimethyl-acetamid aufgenommen und bei 20° C 1,66 g (15 mMol) 4-Fluor-2-picolin in 2 ml N,N-Dimethylacetamid zugetropft. Dann wird 4 Stunden auf 135 - 140° C erhitzt, das Reaktionsgemisch mit Wasser versetzt, mit Dichlormethan extrahiert. Das ölige Rohprodukt wird mit Ethylacetat/Toluol (5:1) an Kieselgel chromatographiert. ($R_f$ = 0,4).

b) 4,3 g (13,3 mMol) des Produkts aus Bsp. 83 a) werden in Dichlormethan bei 20° C unter Rühren mit 2,7 g (13,3 mMol) 85 %iger m-Chlorperbenzoesäure oxidiert. Die organische Phase wird nach 2 Stunden mit gesättigter wäßriger $NaHCO_3$-Lösung ausgeschüttelt, getrocknet und eingeengt, öliges Produkt, $R_f$ - (Ethylacetat/Methanol = 8:1) = 0,08.

**Beispiel 84**

4-(3,4-Dichlorphenoxy)-2-picolin-N-oxid, Fp. 125 - 127° C (aus Petrolether), Herstellung analog Beispiel 73

**Beispiel 85**

4-(3,4-Dichlorphenoxy)-2-hydroxymethyl-pyridin, Fp. 103 - 105 °C (aus Diisopropylether)

**Beispiel 86**

4-(3,4-Dichlorphenoxy)-2-chlormethylpyridin-hydrochlorid, Fp. 173 - 175 °C (aus Diisopropylether)

**Beispiel 87**

2-[4-(3,4-Dichlorphenoxy)-2-picolylmercapto]-1H-thieno-[3,4-d]imidazol, Fp. 161 - 163 °C (aus Ethylacetat)

**Beispiel 88**

2-[4-(3,4-Dichlorphenoxy)-2-picolylsulfinyl]-1H-thieno-[3,4-d]imidazol, Fp. 116 °C (Zers.; aus Toluol)

In analoger Weise lassen sich die folgenden Verbindungen der Formel

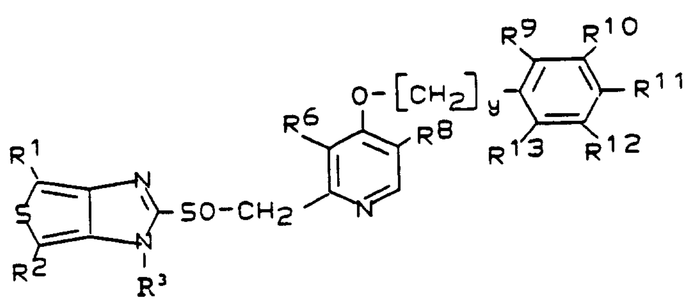

herstellen:

y = 1:

| $R^1$ | $R^2$ | $R^3$ | $R^6$ | $R^8$ | $R^9$ | $R^{10}$ | $R^{11}$ | $R^{12}$ | $R^{13}$ |
|---|---|---|---|---|---|---|---|---|---|
| H | H | H | H | H | F | F | F | F | F |
| H | H | H | H | H | H | $NO_2$ | H | H | H |
| H | H | H | H | H | Cl | H | $CF_3$ | H | H |
| H | H | H | H | H | H | H | CN | H | H |
| H | H | H | H | H | H | Cl | Cl | H | H |
| H | H | H | $CH_3$ | H | H | H | $CF_3$ | H | H |
| H | H | H | $CH_3$ | H | H | H | Cl | H | H |
| H | H | H | $CH_3$ | H | H | Cl | H | Cl | H |
| H | H | H | $CH_3$ | H | H | H | F | H | H |
| H | H | H | $CH_3$ | H | H | Cl | Cl | H | H |
| H | H | H | H | $CH_3$ | H | H | $CF_3$ | H | H |
| H | H | H | H | $CH_3$ | H | Cl | H | Cl | H |
| H | H | H | H | $CH_3$ | H | H | F | H | H |
| H | H | H | H | $CH_3$ | H | H | Cl | H | H |
| $CH_3$ | $CH_3$ | H | H | H | H | H | $CF_3$ | H | H |
| $CH_3$ | $CH_3$ | H | H | H | H | H | F | H | H |
| $CH_3$ | $CH_3$ | H | H | H | H | H | Cl | H | H |
| $CH_3$ | $CH_3$ | H | H | H | H | Cl | H | Cl | H |
| $CH_3$ | $CH_3$ | H | H | H | H | Cl | Cl | H | H |
| $CH_3$ | $CH_3$ | H | $CH_3$ | H | H | H | $CF_3$ | H | H |
| $CH_3$ | $CH_3$ | H | $CH_3$ | H | H | H | F | H | H |
| $CH_3$ | $CH_3$ | H | $CH_3$ | H | H | Cl | Cl | H | H |
| H | H | $CH_2OAc$ | H | H | H | H | $CF_3$ | H | H |
| H | H | $(CH_3)CHOAc$ | H | H | H | H | $CF_3$ | H | H |
| H | H | $CH_2OAc$ | H | H | H | H | F | H | H |
| H | H | $CH_2OAc$ | $CH_3$ | H | H | H | F | H | H |

y = 1;

| $R^1$ | $R^2$ | $R^3$ | $R^6$ | $R^8$ | $R^9$ | $R^{10}$ | $R^{11}$ | $R^{12}$ | $R^{13}$ |
|---|---|---|---|---|---|---|---|---|---|
| H | H | $CH_2OAc$ | H | H | H | H | Cl | H | H |
| H | H | $CH_2OCOOEt$ | H | H | H | Cl | Cl | H | H |
| H | H | $CH_2OCOOEt$ | H | H | H | H | $CF_3$ | H | H |
| H | H | $CH_2OCOOEt$ | H | H | H | H | F | H | H |
| $OCH_2CF_3$ | H | H | H | H | H | H | $CF_3$ | H | H |
| $OCH_2CF_3$ | H | H | H | H | H | H | F | H | H |
| $OCH_2CF_3$ | H | H | H | H | H | H | Cl | H | H |
| $OCH_2CF_3$ | H | H | H | H | H | Cl | Cl | H | H |
| $OCH_2CF_3$ | H | H | $CH_3$ | H | H | H | $CF_3$ | H | H |
| $OCH_2CF_3$ | H | H | $CH_3$ | H | H | H | F | H | H |
| $OCH_2CF_3$ | H | H | $CH_3$ | H | H | H | Cl | H | H |
| $OCH_2CF_3$ | $OCH_2CF_3$ | H | H | H | H | H | $CF_3$ | H | H |
| $OCH_2CF_3$ | $OCH_2CF_3$ | H | H | H | H | H | F | H | H |
| $OCH_2CF_3$ | $OCH_2CF_3$ | H | H | H | H | H | Cl | H | H |
| $OCH_2CF_2CF_3$ | H | H | $CH_3$ | H | H | H | $CF_3$ | H | H |
| $OCH_2CF_2CF_3$ | H | H | $CH_3$ | H | H | H | F | H | H |
| $OCH_2CF_2CF_3$ | H | H | $CH_3$ | H | H | H | Cl | H | H |
| $OCH_2CF_2CF_3$ | $OCH_2CF_2CF_3$ | H | H | H | H | H | $CF_3$ | H | H |
| $OCH_2CF_2CF_3$ | $OCH_3$ | H | H | H | H | H | F | H | H |
| $OCH_2CF_2CF_3$ | $OCH_3$ | H | H | H | H | Cl | Cl | H | H |
| $OCH_2CF_3$ | $OCH_3$ | H | H | H | H | Cl | Cl | H | H |
| $OCH_2CF_3$ | $OCH_3$ | H | H | H | H | H | $CF_3$ | H | H |
| $OCF_2CF_2H$ | H | H | H | H | H | H | $CF_3$ | H | H |
| $OCF_2CF_2H$ | H | H | H | H | H | H | F | H | H |
| $OCF_2CF_2H$ | H | H | H | H | H | H | Cl | H | H |
| $OCF_2CF_2H$ | H | H | $CH_3$ | H | H | H | $CF_3$ | H | H |

y = 1;

| $R^1$ | $R^2$ | $R^3$ | $R^6$ | $R^8$ | $R^9$ | $R^{10}$ | $R^{11}$ | $R^{12}$ | $R^{13}$ |
|---|---|---|---|---|---|---|---|---|---|
| $OCF_2CF_2F$ | H | H | $CH_3$ | H | H | H | Cl | H | H |
| $OCF_2CF_2F$ | H | H | $CH_3$ | H | H | H | F | H | H |
| H | H | $CH_2OCOPh$ | H | H | H | H | $CF_3$ | H | H |
| H | H | $CH_2OCOOBz$ | H | H | H | H | $CF_3$ | H | H |
| H | H | $CH_2OCOOBz$ | H | H | H | H | F | H | H |
| H | H | $CH_2OAc$ | $CH_3$ | H | H | H | $CF_3$ | H | H |

y = 0:

| $R^1$ | $R^2$ | $R^3$ | $R^6$ | $R^8$ | $R^9$ | $R^{10}$ | $R^{11}$ | $R^{12}$ | $R^{13}$ |
|---|---|---|---|---|---|---|---|---|---|
| H | H | H | H | H | F | F | F | F | F |
| H | H | H | $CH_3$ | H | F | F | F | F | F |
| H | H | H | $CH_3$ | $CH_3$ | F | F | F | F | F |
| H | H | H | H | $CH_3$ | F | F | F | F | F |
| $CH_3$ | $CH_3$ | H | H | H | F | F | F | F | F |
| $CH_3$ | $CH_3$ | H | $CH_3$ | H | F | F | F | F | F |
| $CH_3$ | $CH_3$ | H | $CH_3$ | $CH_3$ | F | F | F | F | F |
| $CH_3$ | $CH_3$ | H | H | H | H | $CF_3$ | H | H | H |
| $CH_3$ | $CH_3$ | H | $CH_3$ | H | H | $CF_3$ | H | H | H |
| $CH_3$ | $CH_3$ | H | H | $CH_3$ | H | $CF_3$ | H | H | H |
| H | H | H | $CH_3$ | H | F | H | F | H | H |
| H | H | H | H | $CH_3$ | F | H | F | H | H |
| H | H | H | H | H | H | $CF_3$ | H | $CF_3$ | H |
| H | H | H | $CH_3$ | H | H | $CF_3$ | H | $CF_3$ | H |
| $CH_3$ | $CH_3$ | H | H | H | H | $CF_3$ | H | $CF_3$ | H |
| H | H | H | $OCH_3$ | H | H | $CF_3$ | H | H | H |
| $CH_3$ | $CH_3$ | H | $OCH_3$ | H | H | $CF_3$ | H | H | H |
| H | H | H | H | $OCH_3$ | H | $CF_3$ | H | H | H |
| $CH_3$ | $CH_3$ | H | $OCH_3$ | H | F | H | F | H | H |
| H | H | H | $OCH_3$ | H | H | $CF_3$ | H | $CF_3$ | H |
| H | H | H | $OCH_3$ | H | F | H | F | H | H |
| H | H | H | H | H | Cl | H | Cl | Cl | H |
| H | H | H | $CH_3$ | H | Cl | H | Cl | Cl | H |
| H | H | H | H | $CH_3$ | Cl | H | Cl | Cl | H |
| H | H | H | $OCH_3$ | H | Cl | H | Cl | Cl | H |
| $CH_3$ | $CH_3$ | H | H | H | Cl | H | Cl | Cl | H |
| H | H | H | H | H | F | H | F | F | H |
| H | H | H | H | H | H | Cl | H | Cl | H |
| H | H | H | $OCH_3$ | H | H | Cl | H | Cl | H |
| $CH_3$ | $CH_3$ | H | H | H | H | Cl | H | Cl | H |

y = 0:

| $R^1$ | $R^2$ | $R^3$ | $R^6$ | $R^8$ | $R^9$ | $R^{10}$ | $R^{11}$ | $R^{12}$ | $R^{13}$ |
|---|---|---|---|---|---|---|---|---|---|
| H | H | H | H | $OCH_2CF_3$ | H | H | F | H | H |
| H | H | H | H | $OCH_2CF_3$ | H | F | H | F | H |
| H | H | H | H | $OCH_2CF_3$ | H | $CF_3$ | H | H | H |
| H | H | H | H | $OCH_2CF_3$ | F | H | F | H | H |
| H | H | H | H | $OCF_2CF_2H$ | F | H | F | H | H |
| H | H | H | H | $OCF_2CF_2H$ | H | $CF_3$ | H | H | H |
| H | H | H | $OCH_3$ | H | H | H | F | H | H |
| $CH_3$ | $CH_3$ | H | $OCH_3$ | H | H | H | F | H | H |
| H | H | H | $OCHF_2$ | H | H | H | F | H | H |
| H | H | H | H | H | H | H | $CF_3$ | H | H |
| H | H | H | $CH_3$ | H | H | $CF_3$ | H | $CF_3$ | H |
| $CH_3$ | $CH_3$ | H | $CH_3$ | H | H | $CF_3$ | H | $CF_3$ | H |
| $CH_3$ | $CH_3$ | H | H | H | H | H | $CF_3$ | H | H |
| H | H | H | $OCH_3$ | H | H | H | F | H | H |
| $CH_3$ | $CH_3$ | H | H | H | H | H | F | H | H |
| $CH_3$ | $CH_3$ | H | H | H | H | Cl | Cl | H | H |
| H | H | H | $CH_3$ | H | H | Cl | Cl | H | H |
| $CH_3$ | $CH_3$ | H | H | H | H | H | Cl | H | H |
| H | H | H | $CH_3$ | H | H | H | Cl | H | H |
| H | H | H | $CH_3$ | H | H | H | F | H | H |
| H | H | H | $CH_3$ | H | H | H | $CF_3$ | H | H |
| $CH_3$ | $CH_3$ | H | H | H | H | H | $CF_3$ | H | H |

**Patentansprüche**

**Patentansprüche für folgende Vertragsstaaten : AT, BE, CH, DE, FR, GB, IT, LI, LU, NL, SE**

**1.** Verbindung der Formel I

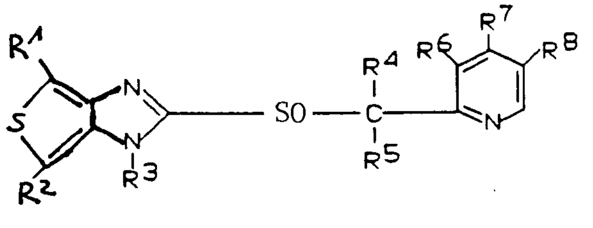

(I)

in welcher

$R^1$ und $R^2$ gleich oder verschieden sind und Wasserstoff, Halogen, $(C_1\text{-}C_3)$-Alkyl, $(C_1\text{-}C_4)$-Alkoxy,

-O-$[CH_2-]_xC_fH_{(2f+1-g)}F_g$, $(C_1-C_4)$-Alkoxycarbonyl,

$R^3$ Wasserstoff, $(C_1-C_6)$-Alkanoyl, $(C_1-C_6)$-Alkylcarbamoyl, $(C_1-C_6)$-Alkoxycarbonyl, Benzyloxycarbonyl oder eine andere physiologisch verträgliche, vorzugsweise im sauren Medium und/oder unter physiologischen Bedingungen abspaltbare $N^{im}$-Schutzgruppe wie z. B. $(C_1-C_{10})$Acyloxy-$(C_1-C_6)$-alkyl, vorzugsweise $(C_1-C_{10})$-Alkanoyloxy-$(C_1-C_6)$-alkyl, Benzoyloxy-$(C_1-C_6)$-alkyl, Benzyloxycarbonyloxy-$(C_1-C_6)$-alkyl oder $(C_1-C_6)$-Alkoxycarbonyloxy-$(C_1-C_6)$-alkyl bedeutet,

$R^4$ und $R^5$ Wasserstoff bedeuten,

$R^6$ und $R^8$ gleich oder verschieden sind und Wasserstoff, Halogen, $(C_1-C_3)$-Alkyl, $(C_1-C_3)$-Alkoxy oder -O-$[CH_2-]_xC_fH_{(2f+1-g)Fg}$, bedeuten,

$R^7$ einen ein- oder mehrfach substituierten Benzyloxy-oder Phenoxy- Rest der Formel

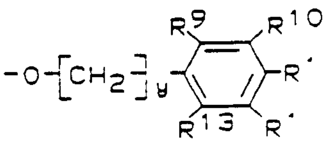

bedeutet,

worin

y 0 oder 1,

f = 1, 2, 3, 4, 5, 6, 7 oder 8 ist,

g = 0, 1 bis (2f + 1) ist,

x = 0, 1, 2 oder 3 ist,

sowie deren physiologisch verträgliche Salze.

**2.** Verbindung der Formel I gemäß Anspruch 1 in welcher

$R^1$ und $R^2$ gleich oder verschieden sind und Wasserstoff oder $(C_1-C_3)$-Alkyl, bedeuten,

$R^3$ wie in Anspruch 1 definiert ist,

$R^4$ und $R^5$ jeweils Wasserstoff bedeuten,

$R^6$ und $R^8$ gleich oder verschieden sind und Wasserstoff, $(C_1-C_3)$-Alkyl oder $(C_1-C_3)$-Alkoxy bedeuten,

$R^7$ einen ein- oder mehrfach substituierten Benzyloxy-oder Phenoxy-Rest bedeutet,

$R^9$, $R^{10}$, $R^{11}$, $R^{12}$ und $R^{13}$ gleich oder verschieden sind und Wasserstoff, Fluor, Chlor, Trifluormethyl bedeuten und die übrigen Reste und Variablen wie in Anspruch 1 definiert sind, sowie deren physiologisch verträgliche Salze.

**3.** 2-[4-(4-Trifluormethylbenzyloxy)-2-picolyl-sulfinyl]-1H thieno[3,4-d]imidazol,
2-[3-Methoxy-4-(4-trifluormethylbenzyloxy)-2-picolylsulfinyl]-1H-thieno[3,4-d]imidazol,
2-[3-Methoxy-4-(4-fluorbenzyloxy)-2-picolylsulfinyl]-1H-thieno[3,4-d]imidazol,
2-[4-(3,5-Bistrifluormethylbenzyloxy)-2-picolylsulfinyl]-1H-thieno[3,4-d]imidazol,
2-[4-(2,4-Difluorphenoxy)-2-picolylsulfinyl]-1H-thieno-[3,4-d]imidazol,
2-[4-(3-Trifluormethylphenoxy)-2-picolylsulfinyl]-1H-thieno[3,4-d]imidazol,
2-[4-(4-Fluorphenoxy)-2-picolylsulfinyl]-1H-thieno[3,4-d] imidazol oder
2-[4-(4-Chlorphenoxy)-2-picolylsulfinyl]-1H-thieno[3,4-d] imidazol
sowie deren physiologisch verträgliche Salze.

**4.** Verfahren zur Herstellung einer Verbindung der Formel I gemäß einem oder mehreren der Ansprüche 1 bis 3, dadurch gekennzeichnet, daß man

a) Verbindungen der Formel II

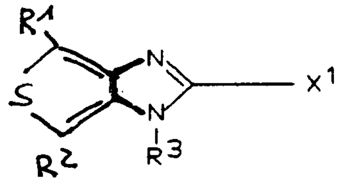

(II)

in welcher $R^1$, $R^2$ und $R^3$ wie oben definiert sind und
$X^1$
i. eine Abgangsgruppe oder
ii. -SH oder $-S^-M^+$ bedeutet,
umsetzt mit Verbindungen der Formel III

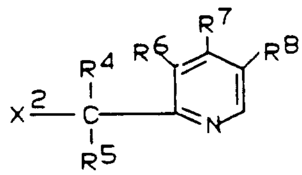

(III)

in welcher $R^4$, $R^5$, $R^6$, $R^7$ und $R^8$ wie oben definiert sind und
$X^2$ im oben genannten Fall i. -SH oder $-S^-M^+$ und im oben genannten Fall ii. vorzugsweise eine Abgangsgruppe oder OH bedeutet, oder
b) Verbindungen der Formel IV,

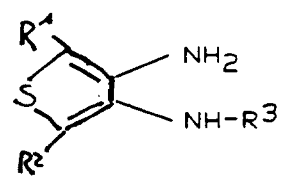

(IV)

in welcher $R^1$, $R^2$ und $R^3$ wie oben definiert sind, umsetzt mit Verbindungen der Formel V

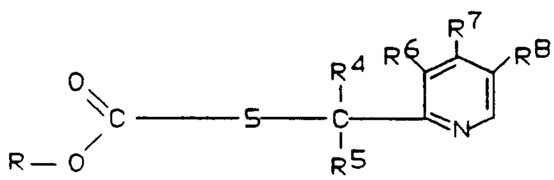

(V)

in welcher $R^4$, $R^5$, $R^6$, $R^7$ und $R^8$ wie oben definiert sind und R für eine veresternde Gruppe steht,
i. in Verbindungen der Formel I (eine) gegebenenfalls vorhandene -S-Gruppe(n) zu(r) -SO-Gruppe(n) oxidiert,
ii. Verbindungen der Formel I, worin $R^3$ für Wasserstoff steht, gewünschtenfalls acyliert, alkyliert oder aralkyliert,
iii. Verbindungen der Formel I, worin $R^3$ nicht Wasserstoff bedeutet, gewünschtenfalls verseift und
iv. Verbindungen der Formel I gewünschtenfalls in ihre physiologisch verträglichen Salze überführt,
wobei zwei oder mehr der Maßnahmen i.-iii. auch in einer anderen als der angegebenen Reihenfolge ausgeführt werden können.

**5.** Verbindung gemäß einem oder mehreren der Ansprüche 1 bis 3 zur Anwendung als Heilmittel.

**6.** Verbindung gemäß Anspruch 5 zur Anwendung als Magensäuresekretionshemmer.

**7.** Pharmazeutische Zubereitung enthaltend eine Verbindung gemäß einem oder mehreren der Ansprüche 1 bis 3, 5 und 6.

**8.** Verfahren zur Herstellung einer Zubereitung gemäß Anspruch 7, dadurch gekennzeichnet, daß man eine Verbindung der Formel I zusammen mit einem physiologisch annehmbaren Träger und gegebenenfalls weiteren Zusatz- oder Hilfsstoffen in eine geeignete Darreichungsform bringt.

**Patentansprüche für folgende Vertragsstaaten : ES, GR**

**1.** Verfahren zur Herstellung einer Verbindung der Formel I

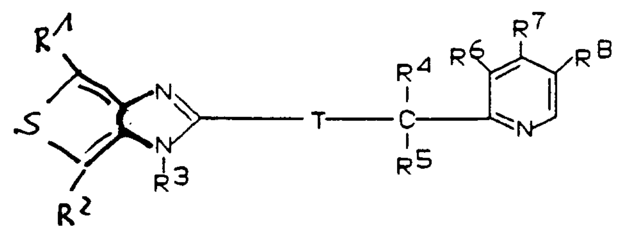

(I)

in welcher

| | |
|---|---|
| $R^1$ und $R^2$ | gleich oder verschieden sind und Wasserstoff, Halogen, $(C_1\text{-}C_3)$-Alkyl, $(C_1\text{-}C_4)$-Alkoxy, $\text{-O-[CH}_2\text{-]}_x C_f H_{(2f+1-g)} F_g$, $(C_1\text{-}C_4)$-Alkoxycarbonyl, |
| $R^3$ | Wasserstoff, $(C_1\text{-}C_6)$-Alkanoyl, $(C_1\text{-}C_6)$-Alkylcarbamoyl, $(C_1\text{-}C_6)$-Alkoxycarbonyl, Benzyloxycarbonyl oder eine andere physiologisch verträgliche, vorzugsweise im sauren Medium und/oder unter physiologischen Bedingungen abspaltbare $N^{im}$-Schutzgruppe wie z. B. $(C_1\text{-}C_{10})$Acyloxy-$(C_1\text{-}C_6)$-alkyl, vorzugsweise $(C_1\text{-}C_{10})$-Alkanoyloxy-$(C_1\text{-}C_6)$-alkyl, Benzoyloxy-$(C_1\text{-}C_6)$-alkyl, Benzyloxycarbonyloxy-$(C_1\text{-}C_6)$-alkyl oder $(C_1\text{-}C_6)$-Alkoxycarbonyloxy-$(C_1\text{-}C_6)$-alkyl bedeutet, |
| $R^4$ und $R^5$ | Wasserstoff bedeuten, |
| $R^6$ und $R^8$ | gleich oder verschieden sind und Wasserstoff, Halogen, $(C_1\text{-}C_3)$-Alkyl, $(C_1\text{-}C_3)$-Alkoxy oder $\text{-O-[CH}_2\text{-]}_x C_f H_{(2f+1-g)Fg}$, bedeuten, |
| $R^7$ | einen ein- oder mehrfach substituierten Benzyloxy-oder Phenoxy- Rest der Formel |

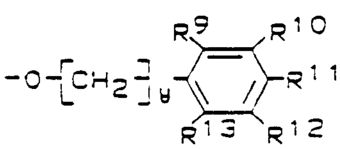

bedeutet,

worin

| | |
|---|---|
| y | 0 oder 1, |
| f = | 1, 2, 3, 4, 5, 6, 7 oder 8 ist, |
| g = | 0, 1 bis (2f + 1) ist, |
| x = | 0, 1, 2 oder 3 ist, |

oder deren physiologisch verträglichen Salzen, dadurch gekennzeichnet, daß man

a) Verbindungen der Formel II

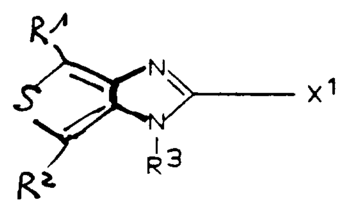

(II)

in welcher $R^1$, $R^2$ und $R^3$ wie oben definiert sind und

$X^1$

i. eine Abgangsgruppe oder

ii. -SH oder $-S^-M^+$ bedeutet,

umsetzt mit Verbindungen der Formel III

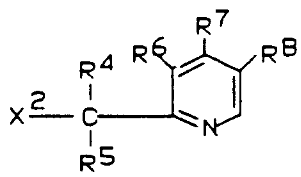

(III)

in welcher $R^4$, $R^5$, $R^6$, $R^7$ und $R^8$ wie oben definiert sind und

$X^2$ im oben genannten Fall i. -SH oder $-S^-M^+$ und im oben genannten Fall ii. vorzugsweise eine Abgangsgruppe oder OH bedeutet, oder

b) Verbindungen der Formel IV,

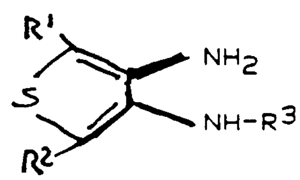

(IV)

in welcher $R^1$, $R^2$ und $R^3$ wie oben definiert sind, umsetzt mit Verbindungen der Formel V

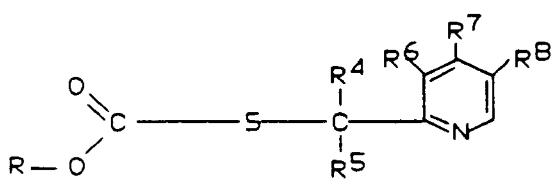

(V)

in welcher $R^4$, $R^5$, $R^6$, $R^7$ und $R^8$ wie oben definiert sind und R für eine veresternde Gruppe steht,

i. in Verbindungen der Formel I (eine) gegebenenfalls vorhandene -S-Gruppe(n) zu(r) -SO-Gruppe(n) oxidiert,

ii. Verbindungen der Formel I, worin $R^3$ für Wasserstoff steht, gewünschtenfalls acyliert, alkyliert oder aralkyliert,

iii. Verbindungen der Formel I, worin $R^3$ nicht Wasserstoff bedeutet, gewünschtenfalls verseift und

iv. Verbindungen der Formel I gewünschtenfalls in ihre physiologisch verträglichen Salze überführt,

wobei zwei oder mehr der Maßnahmen i.-iii. auch in einer anderen als der angegebenen Reihenfolge ausgeführt werden können.

**2.** Verfahren gemäß Anspruch 1 dadurch gekennzeichnet, daß eine Verbindung der Formel I oder deren physiologisch verträgliches Salz hergestellt wird, in welcher

| | |
|---|---|
| $R^1$ und $R^2$ | gleich oder verschieden sind und Wasserstoff oder $(C_1$-$C_3)$-Alkyl, bedeuten, |
| $R^3$ | wie in Anspruch 1 definiert ist, |
| $R^4$ und $R^5$ | jeweils Wasserstoff bedeuten, |
| $R^6$ und $R^8$ | gleich oder verschieden sind und Wasserstoff, $(C_1$-$C_3)$-Alkyl oder $(C_1$-$C_3)$-Alkoxy bedeuten, |
| $R^7$ | einen ein- oder mehrfach substituierten Benzyloxy-oder Phenoxy-Rest bedeutet, |
| $R^9$, $R^{10}$, $R^{11}$, $R^{12}$ und $R^{13}$ | gleich oder verschieden sind und Wasserstoff, Fluor, Chlor, Trifluormethyl bedeuten und die übrigen Reste und Variablen wie in Anspruch 1 |

definiert sind.

3. Verfahren gemäß einem oder mehrerer der Ansprüche 1 und 2, dadurch gekennzeichnet, daß
2-[4-(4-Trifluormethylbenzyloxy)-2-picolyl-sulfinyl]-1H thieno[3,4-d]imidazol,
2-[3-Methoxy-4-(4-trifluormethylbenzyloxy)-2-picolylsulfinyl]-1H-thieno[3,4-d]imidazol,
2-[3-Methoxy-4-(4-fluorbenzyloxy)-2-picolylsulfinyl]-1H-thieno[3,4-d]imidazol,
2-[4-(3,5-Bistrifluormethylbenzyloxy)-2-picolylsulfinyl]-1H-thieno[3,4-d]imidazol,
2-[4-(2,4-Difluorphenoxy)-2-picolylsulfinyl]-1H-thieno-[3,4-d]imidazol,
2-[4-(3-Trifluormethylphenoxy)-2-picolylsulfinyl]-1H-thieno[3,4-d]imidazol,
2-[4-(4-Fluorphenoxy)-2-picolylsulfinyl]-1H-thieno[3,4-d]imidazol,
2-[4-(4-Chlorphenoxy)-2-picolylsulfinyl]-1H-thieno[3,4-d]imidazol
oder deren physiologisch verträglichen Salze hergestellt werden.

4. Verfahren zur Herstellung einer pharmazeutischen Zubereitung enthaltend eine Verbindung hergestellt gemäß einem oder mehreren der Ansprüche 1 bis 3, dadurch gekennzeichnet, daß man diese zusammen mit einem physiologisch annehmbaren Träger und gegebenenfalls weiteren Zusatz- oder Hilfsstoffen in eine geeignete Darreichungsform bringt.

**Claims**
**Claims for the following Contracting States : AT, BE, CH, DE, FR, GB, IT, LI, LU, NL, SE**

1. A compound of the formula I

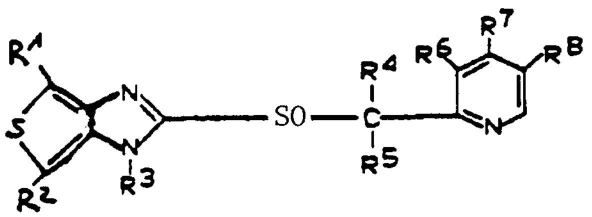

(I)

in which

| | |
|---|---|
| $R^1$ and $R^2$ | are identical or different and are hydrogen, halogen, $(C_1\text{-}C_3)$-alkyl, $(C_1\text{-}C_4)$-alkoxy, $-O-[CH_2-]_xC_fH_{(2f+1-g)}F_g$, $(C_1\text{-}C_4)$-alkoxycarbonyl, |
| $R^3$ | is hydrogen, $(C_1\text{-}C_6)$-alkanoyl, $(C_1\text{-}C_6)$-alkycarbamoyl, $(C_1\text{-}C_6)$-alkoxycarbonyl, benzyloxycarbonyl or another physiologically tolerable $N^{im}$-protecting group which can preferably be cleaved in acidic medium and/or under physiological conditions such as, for example, $(C_1\text{-}C_{10})$-acyloxy-$(C_1\text{-}C_6)$-alkyl, preferably $(C_1\text{-}C_{10})$-alkanoyloxy-$(C_1\text{-}C_6)$-alkyl, benzoyloxy-$(C_1\text{-}C_6)$-alkyl, benzyloxycarbonyloxy-$(C_1\text{-}C_6)$-alkyl or $(C_1\text{-}C_6)$-alkoxycarbonyloxy-$(C_1\text{-}C_6)$-alkyl, |
| $R^4$ and $R^5$ | are hydrogen, |
| $R^6$ and $R^8$ | are identical or different and are hydrogen, halogen, $(C_1\text{-}C_3)$-alkyl, $(C_1\text{-}C_3)$-alkoxy or $-O-[CH_2-]_xC_fH_{(2f+1-g)}F_g$ |
| $R^7$ | is a mono- or polysubstituted benzyloxy or phenoxy radical of the formula |

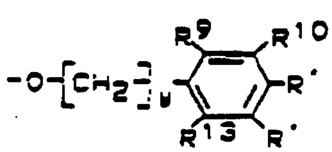

in which

| | |
|---|---|
| y | is 0 or 1, |
| f | is 1, 2, 3, 4, 5, 6, 7 or 8, |
| g | is 0, 1 to (2f + 1), |
| x | is 0, 1, 2 or 3, |

or its physiologically tolerable salts.

**2.** A compound of the formula I as claimed in claim 1, in which

| | |
|---|---|
| $R^1$ and $R^2$ | are identical or different and are hydrogen or $(C_1\text{-}C_3)$-alkyl, |
| $R^3$ | is as defined in claim 1, |
| $R^4$ and $R^5$ | are in each case hydrogen, |
| $R^6$ and $R^8$ | are identical or different and are hydrogen, $(C_1\text{-}C_3)$-alkyl or $(C_1\text{-}C_3)$-alkoxy, |
| $R^7$ | is a mono- or polysubstituted benzyloxy or phenoxy radical, |
| $R^9$, $R^{10}$, $R^{11}$, $R^{12}$ and $R^{13}$ | are identical or different and are hydrogen, fluorine, chlorine, trifluoromethyl and the other radicals and variables are as defined in claim 1, or its physiologically tolerable salts. |

**3.** 2-[4-(4-Trifluoromethylbenzyloxy)-2-picolylsulfinyl]-1H-thieno[3,4-d]imidazole,
2-[3-methoxy-4-(4-trifluoromethylbenzyloxy)-2-picolylsulfinyl]-1H-thieno[3,4-d]imidazole,
2-[3-methoxy-4-(4-fluorobenzyloxy)-2-picolylsulfinyl]-1H-thieno[3,4-d]imidazole,
2-[4-(3,5-bistrifluoromethylbenzyloxy)-2-picolylsulfinyl]-1H-thieno[3,4-d]imidazole,
2-[4-(2,4-difluorophenoxy)-2-picolylsulfinyl]-1H-thieno[3,4-d]imidazole,
2-[4-(3-trifluoromethylphenoxy)-2-picolylsulfinyl]-1H-thieno[3,4-d]imidazole,
2-[4-(4-fluorophenoxy)-2-picolylsulfinyl]-1H-thieno[3,4-d]imidazole or
2-[4-(4-chlorophenoxy)-2-picolylsulfinyl]-1H-thieno-[3,4-d]imidazole
or their physiologically tolerable salts.

**4.** A process for the preparation of a compound of the formula I as claimed in one or more of claims 1 to 3, which comprises

a) reacting compounds of the formula II

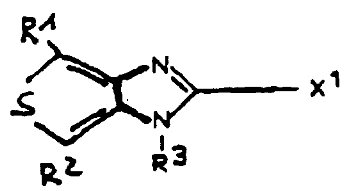

(II)

in which $R^1$, $R^2$ and $R^3$ are as defined above and

$X^1$ is

i. a leaving group or

ii. -SH or $-S^-M^+$,

with compounds of the formula III

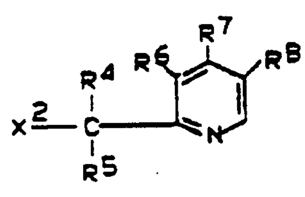

(III)

in which $R^4$, $R^5$, $R^6$, $R^7$ and $R^8$ are as defined above and

$X^2$ in the abovementioned case i. is -SH or $-S^-M^+$ and in the abovementioned case ii. is preferably a leaving group or OH, or

b) reacting compounds of the formula IV

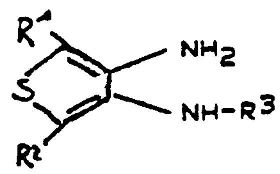

(IV)

in which $R^1$, $R^2$ and $R^3$ are as defined above, with compounds of the formula V

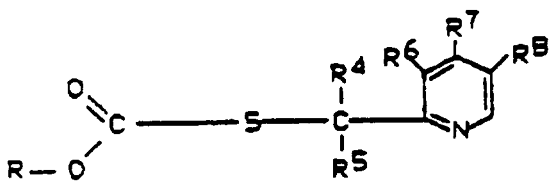

(V)

in which $R^4$, $R^5$, $R^6$, $R^7$ and $R^8$ are as defined above and R is an esterifying group,

i. in compounds of the formula I, oxidizing (an) optionally present -S- group(s) to give (the) -SO- group,

ii. if desired acylating, alkylating or aralkylating compounds of the formula I, in which $R^3$ is hydrogen,

iii. if desired hydrolyzing compounds of the formula I in which $R^3$ is not hydrogen, and

iv. if desired converting compounds of the formula I into their physiologically tolerable salts,

where two or more of the measures i.-iii. can also be carried out in a sequence other than that indicated.

**5.** A compound as claimed in one or more of claims 1 to 3 for use as a medicament.

**6.** A compound as claimed in claim 5 for use as an inhibitor of gastric acid secretion.

**7.** A pharmaceutical preparation containing a compound as claimed in one or more of claims 1 to 3, 5 and 6.

**8.** A process for the production of a preparation as claimed in claim 7, which comprises bringing a compound of the formula I into a suitable administration form together with a physiologically tolerable excipient and, if appropriate, other additives or auxiliaries.

**Claims for the following Contracting States : ES, GR**

**1.** A process for the preparation of a compound of the formula I

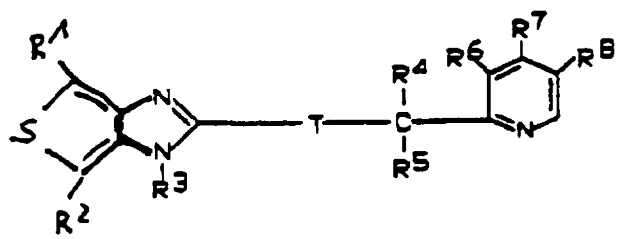

(I)

in which

$R^1$ and $R^2$ are identical or different and are hydrogen, halogen, $(C_1\text{-}C_3)$-alkyl, $(C_1\text{-}C_4)$-alkoxy, $-O-[CH_2-]_xC_fH_{(2f+1-g)}F_g$, $(C_1\text{-}C_4)$-alkoxycarbonyl,

$R^3$ is hydrogen, $(C_1-C_6)$-alkanoyl, $(C_1-C_6)$-alkylcarbamoyl, $(C_1-C_6)$-alkoxycarbonyl, benzyloxycarbonyl or another physiologically tolerable $N^{im}$-protecting group which can preferably be cleaved in acidic medium and/or under physiological conditions such as, for example, $(C_1-C_{10})$-acyloxy-$(C_1-C_6)$-alkyl, preferably$(C_1-C_{10})$-alkanoyloxy-$(C_1-C_6)$-alkyl, benzoyloxy-$(C_1-C_6)$-alkyl, benzyloxycarbonyloxy-$(C_1-C_6)$-alkyl or $(C_1-C_6)$-alkoxycarbonyloxy-$(C_1-C_6)$-alkyl,

$R^4$ and $R^5$ are hydrogen,

$R^6$ and $R^8$ are identical or different and are hydrogen, halogen, $(C_1-C_3)$-alkyl, $(C_1-C_3)$-alkoxy or $-O-[CH_2-]_xC_fH_{(2f+1-g)}F_g$

$R^7$ is a mono- or polysubstituted benzyloxy or phenoxy radical of the formula

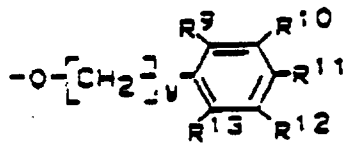

in which

y is 0 or 1,

f is 1, 2, 3, 4, 5, 6, 7 or 8,

g is 0, 1 to (2f + 1),

x is 0, 1, 2 or 3,

or its physiologically tolerable salts,

which comprises

a) reacting compounds of the formula II

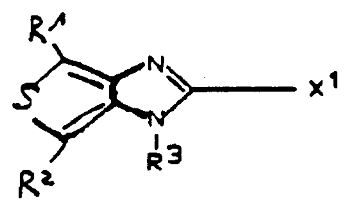

(II)

in which $R^1$, $R^2$ and $R^3$ are as defined above and

$X^1$ is

i. a leaving group or

ii. -SH or $-S^-M^+$,

with compounds of the formula III

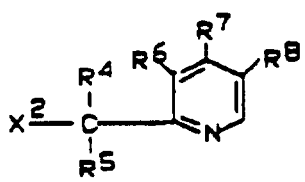

(III)

in which $R^4$, $R^5$, $R^6$, $R^7$ and $R^8$ are as defined above and

$X^2$ in the abovementioned case i. is -SH or $-S^-M^+$ and in the abovementioned case ii. is preferably a leaving group or OH, or

b) reacting compounds of the formula IV

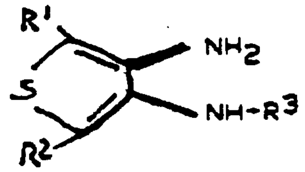

(IV)

in which $R^1$, $R^2$ and $R^3$ are as defined above, with compounds of the formula V

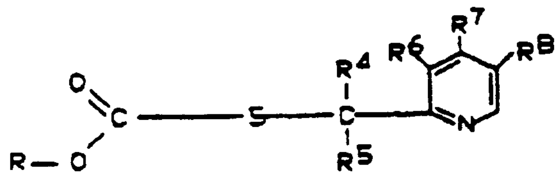

(V)

in which $R^4$, $R^5$, $R^6$, $R^7$ and $R^8$ are as defined above and R is an esterifying group,

i. in compounds of the formula I, oxidizing (an) optionally present -S- group(s) to give (the) -SO- group,

ii. if desired acylating, alkylating or aralkylating compounds of the formula I, in which $R^3$ is hydrogen,

iii. if desired hydrolyzing compounds of the formula I in which $R^3$ is not hydrogen, and

iv. if desired converting compounds of the formula I into their physiologically tolerable salts,

where two or more of the measures i.-iii. can also be carried out in a sequence other than that indicated.

**2.** The process as claimed in claim 1, wherein a compound of the formula I or its physiologically tolerable salt is prepared, in which

| | |
|---|---|
| $R^1$ and $R^2$ | are identical or different and are hydrogen or $(C_1-C_3)$-alkyl, |
| $R^3$ | is as defined in claim 1, |
| $R^4$ and $R^5$ | are in each case hydrogen, |
| $R^6$ and $R^8$ | are identical or different and are hydrogen, $(C_1-C_3)$-alkyl or $(C_1-C_3)$-alkoxy, |
| $R^7$ | is a mono- or polysubstituted benzyloxy or phenoxy radical, |
| $R^9$, $R^{10}$, $R^{11}$, $R^{12}$ and $R^{13}$ | are identical or different and are hydrogen, fluorine, chlorine, trifluoromethyl and the other radicals and variables are as defined in claim 1. |

**3.** The process as claimed in one or more of claims 1 and 2, wherein
2-[4-(4-trifluoromethylbenzyloxy)-2-picolylsulfinyl]-1H-thieno[3,4-d]imidazole,
2-[3-methoxy-4-(4-trifluoromethylbenzyloxy)-2-picolylsulfinyl]-1H-thieno[3,4-d]imidazole,
2-[3-methoxy-4-(4-fluorobenzyloxy)-2-picolylsulfinyl]-1H-thieno[3,4-d]imidazole,
2-[4-(3,5-bistrifluoromethylbenzyloxy)-2-picolylsulfinyl]-1H-thieno[3,4-d]imidazole,
2-[4-(2,4-difluorophenoxy)-2-picolylsulfinyl]-1H-thieno[3,4-d]imidazole,
2-[4-(4-fluorophenoxy)-2-picolylsulfinyl]-1H-thieno[3,4-d]imidazole,
2-[4-(4-chlorophenoxy)-2-picolylsulfinyl]-1H-thieno-[3,4-d]imidazole
or their physiologically tolerable salts are prepared.

**4.** A process for the production of a pharmaceutical preparation containing a compound prepared as claimed in one or more of claims 1 to 3, which comprises bringing this into a suitable administration form together with a physiologically tolerable excipient and, if appropriate, other additives or auxiliaries.

**Revendications**

**Revendications pour les Etats contractants suivants : AT, BE, CH, DE, FR, GB, IT, LI, LU, NL, SE**

**1.** Composé de formule I

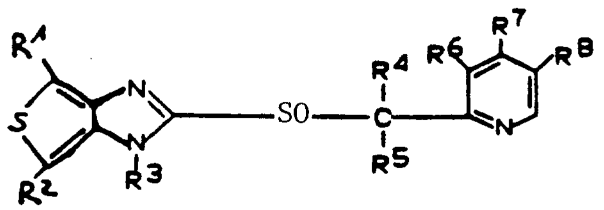

(I)

dans laquelle

$R^1$ et $R^2$ sont identiques ou différents et représentent un atome d'hydrogène ou d'halogène ou un groupe alkyle en $C_1$-$C_3$, alcoxy en $C_1$-$C_4$, $-O-[CH_2-]_x-C_fH_{(2f+1-g)}F_g$ ou alcoxy($C_1$-$C_4$)-carbonyle,

$R^3$ représente un atome d'hydrogène ou un radical alcanoyle en $C_1$-$C_6$, alkyl($C_1$-$C_6$)-carbamoyle, alcoxy($C_1$-$C_6$)-carbonyle, benzyloxycarbonyle, ou un autre groupe protecteur d'azote en fonction imine, physiologiquement acceptable, de préférence séparable en milieu acide et/ou dans des conditions physiologiques, comme par exemple un groupe acyloxy-($C_1$-$C_{10}$)-alkyle($C_1$-$C_6$), de préférence un groupe alcanoyloxy($C_1$-$C_{10}$)-alkyle($C_1$-$C_6$), benzoyloxy-alkyle($C_1$-$C_6$), benzyloxycarbonyloxy-alkyle($C_1$-$C_6$) ou alcoxy($C_1$-$C_6$)-carbonyloxy-alkyle($C_1$-$C_6$);

$R^4$ et $R^5$ représentent chacun un atome d'hydrogène;

$R^6$ et $R^8$ sont identiques ou différents, et représentent un atome d'hydrogène ou d'halogène, ou un groupe alkyle en $C_1$-$C_3$, alcoxy en $C_1$-$C_3$ ou $-O-[CH_2-]_xC_fH_{(2f+1-g)}F_g$;

$R^7$ représente un radical benzyloxy ou phénoxy une ou plusieurs fois substitué, de formule

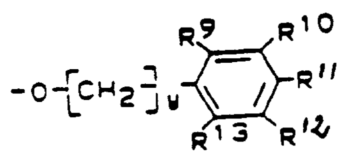

dans laquelle

$R^9$, $R^{10}$, $R^{11}$, $R^{12}$ et $R^{13}$ sont identiques ou différents, et représentent un atome d'hydrogène, de fluor, de chlore ou de brome, ou un groupe trifluorométhyle, cyano, nitro, alcoxy($C_1$-$C_6$)-carbonyle;

et

y est 0 ou 1,

f est 1, 2, 3, 4, 5, 6, 7 ou 8,

g est 0, 1 à (2f + 1) et

x est 0, 1, 2 ou 3,

et sels physiologiquement acceptables de celui-ci.

**2.** Composé de formule I selon la revendication 1, dans lequel

$R^1$ et $R^2$ sont identiques ou différents, et représentent un atome d'hydrogène ou un groupe alkyle en $C_1$-$C_3$,

$R^3$ est tel que défini dans la revendication 1,

$R^4$ et $R^5$ représentent chacun un atome d'hydrogène,

$R^6$ et $R^8$ sont identiques ou différents, et représentent un atome d'hydrogène ou un groupe alkyle en $C_1$-$C_3$ ou alcoxy en $C_1$-$C_3$,

$R^7$ représente un radical benzyloxy ou phénoxy une ou plusieurs fois substitué,

$R^9$, $R^{10}$, $R^{11}$, $R^{12}$ et $R^{13}$ sont identiques ou différents, et représentent un atome d'hydrogène, de

fluor ou de chlore ou le groupe trifluorométhyle,
et les autres radicaux et variables sont tels que définis dans la revendication 1, et sels physiologiquement acceptables de celui-ci.

3. 2-[4-(4-trifluorométhylbenzyloxy)-2-picolylsulfinyl]-1H-thiéno[3,4-d]imidazole,
2-[3-méthoxy-4-(4-trifluorométhylbenzyloxy)-2-picolylsulfinyl]-1H-thiéno[3,4-d]imidazole,
2-[3-méthoxy-4-(4-fluorobenzyloxy)-2-picolylsulfinyl]-1H-thiéno[3,4-d]imidazole,
2-[4-(3,5-bis-trifluorométhylbenzyloxy)-2-picolylsulfinyl]-1H-thiéno[3,4-d]imidazole,
2-[4-(2,4-difluorophénoxy)-2-picolylsulfinyl]-1H-thiéno-[3,4-d]imidazole,
2-[4-(3-trifluorométhylphénoxy)-2-picolylsulfinyl-1H-thiéno[3,4-d]imidazole,
2-[4-(4-fluorophénoxy)-2-picolylsulfinyl]-1H-thiéno-[3,4-d]imidazole,
2-[4-(4-chlorophénoxy)-2-picolylsulfinyl]-1H-thiéno-[3,4-d]imidazole,
et sels physiologiquement acceptables de ceux-ci.

4. Procédé pour la préparation d'un composé de formule I selon une ou plusieurs des revendications 1 à 3,
caractérisé en ce que
a) on fait réagir des composés de formule II

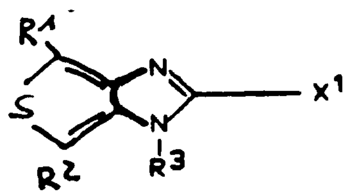

(II)

dans laquelle $R^1$, $R^2$ et $R^3$ sont tels que définis plus haut, et $X^1$ représente
I. un groupe partant ou
II. -SH ou $-S^-M^+$,
avec des composés de formule III

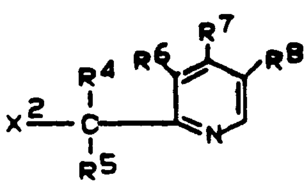

(III)

dans laquelle $R^4$, $R^5$, $R^6$, $R^7$ et $R^8$ sont tels que définis plus haut, et
$X^2$ représente
dans le cas I. mentionné ci-dessus, -SH ou $-S^-M^+$, et dans le cas II. mentionné ci-dessus, de préférence un groupe partant ou OH, ou
b) on fait réagir des composés de formule IV

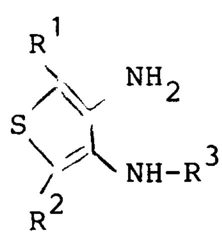

(IV),

dans laquelle $R^1$, $R^2$ et $R^3$ sont tels que définis plus haut,
avec des composés de formule V

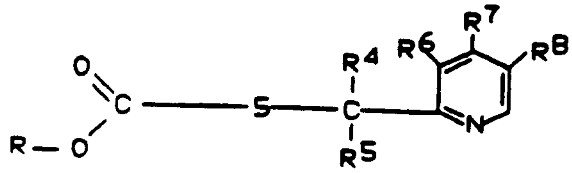

(V)

dans laquelle $R^4$, $R^5$, $R^6$, $R^7$ et $R^8$ sont tels que définis plus haut, et R représente un groupe estérifiant, et

I. dans des composés de formule I, on oxyde en groupe(s) -SO un(des) atome(s) de S éventuellement présent(s);

II. on soumet si on le désire à une acylation, alkylation ou aralkylation des composés de formule I dans lesquels $R^3$ représente un atome d'hydrogène;

III. on saponifie si on le désire des composés de formule I dans lesquels $R^3$ ne représente pas un atome d'hydrogène; et

IV. on transforme si on le désire des composés de formule I en leurs sels physiologiquement acceptables;

deux des opérations I-III ou plus pouvant également être effectuées dans un ordre différent de celui indiqué.

**5.** Composé selon une ou plusieurs des revendications 1 à 3, pour utilisation en tant que médicament.

**6.** Composé selon la revendication 5, pour utilisation en tant qu'inhibiteur de la sécrétion d'acide gastrique.

**7.** Composition pharmaceutique contenant un composé selon une ou plusieurs des revendications 1 à 3, 5 et 6.

**8.** Procédé pour la préparation d'une composition selon la revendication 7, caractérisé en ce que l'on met sous une forme d'administration appropriée un composé de formule I, conjointement avec un véhicule physiologiquement acceptable et éventuellement d'autres additifs ou adjuvants.

**Revendications pour les Etats contractants suivants : ES, GR**

**1.** Procédé pour la préparation d'un composé de formule I

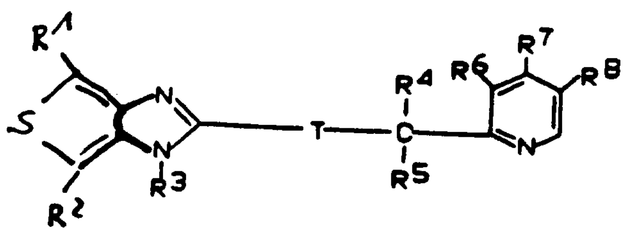

(I)

dans laquelle

| | |
|---|---|
| $R^1$ et $R^2$ | sont identiques ou différents et représentent un atome d'hydrogène ou d'halogène ou un groupe alkyle en $C_1$-$C_3$, alcoxy en $C_1$-$C_4$, -O-$[CH_2-]_x$-$C_fH_{(2f+1-g)}F_g$ ou alcoxy($C_1$-$C_4$)-carbonyle, |
| $R^3$ | représente un atome d'hydrogène ou un radical alcanoyle en $C_1$-$C_6$, alkyl($C_1$-$C_6$)-carbamoyle, alcoxy($C_1$-$C_6$)-carbonyle, benzyloxycarbonyle, ou un autre groupe protecteur d'azote en fonction imine, physiologiquement acceptable, de préférence séparable en milieu acide et/ou dans des conditions physiologiques, comme par exemple un groupe acyloxy-($C_1$-$C_{10}$)-alkyle($C_1$-$C_6$), de préférence un groupe alcanoyloxy($C_1$-$C_{10}$)-alkyle-($C_1$-$C_6$), benzoyloxy-alkyle($C_1$-$C_6$), benzyloxycarbonyloxy-alkyle($C_1$-$C_6$) ou alcoxy($C_1$-$C_6$)-carbonyloxy-alkyle($C_1$-$C_6$); |

$R^4$ et $R^5$ représentent chacun un atome d'hydrogène;
$R^6$ et $R^8$ sont identiques ou différents, et représentent un atome d'hydrogène ou d'halogène, ou un groupe alkyle en $C_1$-$C_3$, alcoxy en $C_1$-$C_3$ ou -O-[$CH_2$-]$_xC_fH_{(2f+1-g)}F_g$;
$R^7$ représente un radical benzyloxy ou phénoxy une ou plusieurs fois substitué, de formule

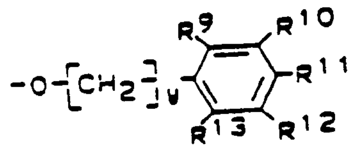

dans laquelle
$R^9$, $R^{10}$, $R^{11}$, $R^{12}$ et $R^{13}$ sont identiques ou différents, et représentent un atome d'hydrogène, de fluor, de chlore ou de brome, ou un groupe trifluorométhyle, cyano, nitro, alcoxy($C_1$-$C_6$)-carbonyle;

et
y est 0 ou 1,
f est 1, 2, 3, 4, 5, 6, 7 ou 8,
g est 0, 1 à (2f+1) et
x est 0, 1, 2 ou 3,

ou de ses sels physiologiquement acceptables,
caractérisé en ce que
a) on fait réagir des composés de formule II

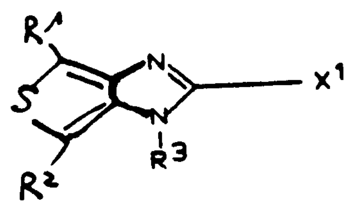

(II)

dans laquelle $R^1$, $R^2$ et $R^3$ sont tels que définis plus haut, et $X^1$ représente
I. un groupe partant ou
II. -SH ou $-S^-M^+$,
avec des composés de formule III

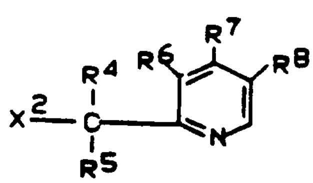

(III)

dans laquelle $R^4$, $R^5$, $R^6$, $R^7$ et $R^8$ sont tels que définis plus haut, et
$X^2$ représente
dans le cas I. mentionné ci-dessus, -SH ou $-S^-M^+$, et dans le cas II. mentionné ci-dessus, de préférence un groupe partant ou OH, ou

b) on fait réagir des composés de formule IV

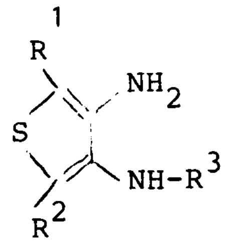

(IV),

dans laquelle $R^1$, $R^2$ et $R^3$ sont tels que définis plus haut,
avec des composés de formule V

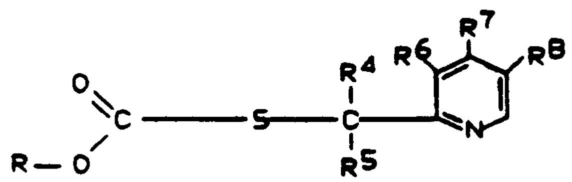

(V)

dans laquelle $R^4$, $R^5$, $R^6$, $R^7$ et $R^8$ sont tels que définis plus haut, et R représente un groupe estérifiant, et

I. dans des composés de formule I, on oxyde en groupe(s) -SO un(des) atome(s) de S éventuellement présent(s);

II. on soumet si on le désire à une acylation, alkylation ou aralkylation des composés de formule I dans lesquels $R^3$ représente un atome d'hydrogène;

III. on saponifie si on le désire des composés de formule I dans lesquels $R^3$ ne représente pas un atome d'hydrogène; et

IV. on transforme si on le désire des composés de formule I en leurs sels physiologiquement acceptables;

deux des opérations I-III ou plus pouvant également être effectuées dans un ordre différent de celui indiqué.

**2.** Procédé selon la revendication 1, caractérisé en ce que l'on prépare un composé de formule I ou un sel physiologiquement acceptable de celui-ci, dans lequel

| | |
|---|---|
| $R^1$ et $R^2$ | sont identiques ou différents, et représentent un atome d'hydrogène ou un groupe alkyle en $C_1$-$C_3$, |
| $R^3$ | est tel que défini dans la revendication 1, |
| $R^4$ et $R^5$ | représentent chacun un atome d'hydrogène, |
| $R^6$ et $R^8$ | sont identiques ou différents, et représentent un atome d'hydrogène ou un groupe alkyle en $C_1$-$C_3$ ou alcoxy en $C_1$-$C_3$, |
| $R^7$ | représente un radical benzyloxy ou phénoxy une ou plusieurs fois substitué, |
| $R^9$, $R^{10}$, $R^{11}$, $R^{12}$ et $R^{13}$ | sont identiques ou différents, et représentent un atome d'hydrogène, de fluor ou de chlore ou le groupe trifluorométhyle, |

et les autres radicaux et variables sont tels que définis dans la revendication 1.

**3.** Procédé selon une ou plusieurs des revendications 1 et 2, caractérisé en ce que l'on prépare
le 2-[4-(4-trifluorométhylbenzyloxy)-2-picolyl- sulfinyl]-1H-thiéno[3,4-d]imidazole,
le 2-[3-méthoxy-4-(4-trifluorométhylbenzyloxy)-2-picolylsulfinyl]-1H-thiéno[3,4-d]imidazole,
le 2-[3-méthoxy-4-(4-fluorobenzyloxy)-2-picolylsulfinyl]-1H-thiéno[3,4-d]imidazole,
le 2-[4-(3,5-bis-trifluorométhylbenzyloxy)-2-picolylsulfinyl]-1H-thiéno[3,4-d]imidazole,
le 2-[4-(2,4-difluorophénoxy)-2-picolylsulfinyl]-1H-thiéno-[3,4-d]imidazole,
le 2-[4-(3-trifluorométhylphénoxy)-2-picolylsulfinyl-1H-thiéno[3,4-d]imidazole,
le 2-[4-(4-fluorophénoxy)-2-picolylsulfinyl]-1H-thiéno-[3,4-d]imidazole,
le 2-[4-(4-chlorophénoxy)-2-picolylsulfinyl]-1H-thiéno-[3,4-d]imidazole,

ou leurs sels physiologiquement acceptables.

**4.** Procédé pour la préparation d'une composition pharmaceutique contenant un composé préparé selon une ou plusieurs des revendications 1 à 3, caractérisé en ce qu'on le met sous une forme d'administration appropriée, conjointement avec un véhicule physiologiquement acceptable et éventuellement d'autres additifs ou adjuvants.